# EUROPEAN PATENT APPLICATION

(11) **EP 4 471 053 A1**
(43) Date of publication of application: **04.12.2024**
(21) Application number: 23746336.9
(22) Date of filing: 20.01.2023
(51) Int. Cl.: C07K 14/62, A61K 38/28, A61P 3/10

(54) **ACYLATED INSULIN**

(30) Priority: 28.01.2022 CN 202210109329
(71) Applicant: Gan & Lee Pharmaceuticals Co., Ltd., Beijing 101109 (CN)
(72) Inventor: GAN, Zhongru, Beijing 101109 (CN); CHEN, Wei, Beijing 101109 (CN); ZHANG, Yining, Beijing 101109 (CN); BI, Juanjuan, Beijing 101109 (CN); XUE, Fangkai, Beijing 101109 (CN); NIU, Jianghong, Beijing 101109 (CN)
(74) Representative: Kraus & Lederer PartGmbB
(86) International application number: PCT/CN2023/073399
(87) International publication number: WO 2023/143458

(57) **Abstract**

Provided are a new acylated insulin, a pharmaceutical preparation thereof, a pharmaceutical composition thereof containing a long-acting GLP-1 compound, and the medical use of the acylated insulin, the pharmaceutical preparation and the pharmaceutical composition.

## Description

### TECHNICAL FIELD

The present invention relates to the field of therapeutic peptides, in particular to novel acylated insulins, pharmaceutical formulations thereof, pharmaceutical compositions thereof with long-acting GLP-1 compounds, and pharmaceutical uses of the acylated insulins, pharmaceutical formulations and pharmaceutical compositions.

### BACKGROUND

Insulin is a polypeptide hormone secreted by β cells of the pancreas. Insulin consists of two polypeptide chains named as A chain and B chain, which are linked together by two inter-chain disulfide bonds. In human, porcine and bovine insulin, the A chain and the B chain contain 21 and 30 amino acid residues, respectively. However, from species to species, there are variations among the amino acid residues presented in different positions in the 2 chains. The widespread use of genetic engineering has made it possible to prepare analogues of natural insulins by substitution, deletion and addition of one or more amino acid residues.

Insulin can be used to treat diabetes and diseases associated with or resulting from it, and it is essential in maintaining normal metabolic regulation. However, natural insulins such as human insulins have a relatively short duration of action, which necessitates frequent injections by the patients and causes a lot of injection-related discomfort in the patient. Therefore, there is continuing effort to obtain insulin derivatives or analogues with longer duration of action, lower frequency of injection and improved drug effect to ameliorate the inconvenience and discomfort associated with high frequency of insulin injection or higher concentration of insulin injection.

WO1995007931A1 has disclosed insulin detemir, a commercially available long-acting insulin, which has a molecular structural feature that threonine at position 30 of the B chain of human insulin is deleted and a 14-carbon fatty monoacid is connected to lysine residue at position 29 of the B chain.WO2005012347A2 has disclosed insulin degludec, another long-acting insulin, which is a novel super long-acting insulin with longer duration of action than insulin detemir and has a molecular structural feature that threonine at position 30 of the B chain of human insulin is deleted and a 16-carbon fatty diacid side chain is connected to lysine residue at position B29 via a glutamic acid molecule. CN101573133B and WO2009/010428 disclose PEGylated extended insulin, which has a longer duration of action compared to a conventional unmodified insulin. WO2013086927A1 and WO2018/024186 have disclosed a long-acting acylated derivative of human insulin analogue.

However, to date, no basal insulin product whose subcutaneous injection frequency is less than once daily combined good medicinal effects has been approved for sale.

Thus, there is still a need for insulin derivatives or analogues with longer duration of action, lower frequency of administration and better effect or efficacy in vivo and superior physicochemical properties compared to the insulins already on the market (e.g., insulin degludec) or the known insulin derivatives.

### SUMMARY

The present invention provides novel acylated insulins. Through a large number of experiments, the inventors unexpectedly found that the novel acylated insulin has unexpectedly and significantly increased longer duration of action, longer in *vivo* half-life, better in *vivo* efficacy, better bioavailability, better safety, and more satisfactory physical stability, chemical stability, and solubility compared with the commercially available insulin degludec (trade name "Tresiba") or some other insulin derivatives.

The first aspect of the present invention provides acylated insulin of formula (A), or a pharmaceutically acceptable salt, amide or ester thereof: III-(II)ₘ-(I)ₙ-ins (A),
wherein, ins is an insulin parent of the acylated insulin, and III-(II)ₘ-(I)ₙ is an acyl moiety of the acylated insulin;
the insulin parent is A14E, B16H, B25H, desB30 human insulin (SEQ ID NO: 1 and SEQ ID NO: 2, representing A chain and B chain respectively),
or A14E, B16E, B25H, desB30 human insulin (SEQ ID NO:3 and SEQ ID NO:4, representing the A chain and the B chain respectively), the acyl moiety is linked to the ε amino group of the lysine residue at the B29 position of the insulin parent;
   I is a neutral, alkylene glycol-containing amino acid residue;
   II is an acidic amino acid residue;
   III is a fatty diacid comprising 22, 23, 24, 25, or 26 carbon atoms, wherein formally, a hydroxyl group has been removed from one of the carboxyl groups in the fatty diacid;

   III, II, and I are linked by an amide bond, and the order of II and I represented in the formula (A) can be interchanged independently;
   m is 1, 2, 3, 4 or 5; and
   n is an integer of 3 or 4.

Through a large number of experiments, the inventors unexpectedly found that the acylated insulin of the present invention enables the insulin derivatives of the present invention to have an unexpectedly longer action time, a half-life in *vivo,* higher bioavailability, better chemical stability than existing insulin derivatives, more importantly, at the same time, the acylated insulin of the present invention also has better *in vivo* potency, efficacy or drug effect, which can enable patients to reduce the discomfort caused by the injection of high-concentration insulin.

In some embodiments, I is: -HN-(CH₂)₂-O-(CH₂)₂-O-CH₂-CO-, -HN-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₃-O-(CH₂)₄-O-(CH₂)₃-NH-CO-, -HN-(CH₂)₃-O-(CH₂)₄-O-(CH₂)₃-NH-CO-CH₂-O-CH₂-CO-, -HN-(CH₂)₃-O-(CH₂)₄-O-(CH₂)₃-NH-CO-(CH₂)₂-CO-, -HN-(CH₂)₂-O-(CH₂)₂-O-CH₂-CO-CH₂-O-CH₂-CO-, -HN-(CH₂)₃-O-(CH₂)₂-O-(CH₂)₂-O-(CH₂)₃-NH-CO-(CH₂)₂-CO-, -HN-(CH₂)₃-O-(CH₂)₂-O-(CH₂)₂-O-(CH₂)₃-NH-CO-CH₂-O-CH₂-CO-, -HN-(CH₂)₂-O-(CH₂)₂-O-(CH₂)₂-NH-CO-(CH₂)₂-CO-, - HN-(CH₂)₂-O-(CH₂)₂-O-(CH₂)₂-NH-CO-CH₂-O-CH₂-CO-, -HN-(CH₂)₃-O-(CH₂)₂-O-(CH₂)₂-O-(CH₂)₃-NH-CO-CH₂-O-CH₂-CO-, -HN-(CH₂)₃-O-(CH₂)₃-O-CH₂-CO-, or -HN-(CH₂)₄-O-(CH₂)₄-O-CH₂-CO-; preferably, III is HOOC-(CH₂)₂₀-CO-, HOOC-(CH₂)₂₁-CO-, or HOOC-(CH₂)₂₂-CO-, preferably I is -HN-(CH₂)₂-O-(CH₂)₂-O-CH₂-CO.

In some embodiments, II is an amino acid residue selected from the group consisting of γGlu, αGlu, βAsp, αAsp, γ-D-Glu, α-D-Glu, β-D-Asp and α-D-Asp, preferably, II is γGlu.

In some embodiments, III is a fatty diacid comprising 22, 23 or 24 carbon atoms, wherein formally a hydroxyl group has been removed from one of the carboxyl groups of the fatty diacid; preferably, III is HOOC-(CH₂)₂₀-CO-.

In some embodiments, n is 3. In other embodiments m is 1 or 2.

In some embodiments, the acylated insulin is selected from the group consisting of the following insulins: A14E, B16H, B25H, B29K(N(ε)-docosanedioyl-yGlu-3xOEG), desB30 human insulin; A14E, B16H, B25H, B29K(N(ε)-docosanedioyl-γGlu-4xOEG), desB30 human insulin; A14E, B16H, B25H, B29K(N(ε)-tricosanedioyl-γGlu-3xOEG), desB30 human insulin; A14E, B16H, B25H, B29K(N(ε)-tricosanedioyl-γGlu-4xOEG), desB30 human insulin; A14E, B16H, B25H, B29K(N(ε)-tetracosanedioyl-yGlu-3xOEG), desB30 human insulin; A14E, B16H, B25H, B29K (N(ε)-tetracosanedioyl-yGlu-4xOEG), desB30 human insulin; A14E, B16E, B25H, B29K (N(ε)-docosanedioyl-γGlu-3xOEG), desB30 human insulin; A14E, B16E, B25H, B29K (N(ε)-docosanedioyl-γGlu-4xOEG), desB30 human insulin; A14E, B16E, B25H, B29K(N(ε)-tricosanedioyl-γGlu-3xOEG), desB30 human insulin; A14E, B16E, B25H, B29K (N(ε)-tricosanedioyl-yGlu-4xOEG), desB30 human insulin; A14E, B16E, B25H, B29K(N(ε)-tetracosanedioyl-γGlu-3xOEG), desB30 human insulin; and A14E, B16E, B25H, B29K (N(ε)-tetracosanedioyl-γGlu-4xOEG), desB30 human insulin.

In some embodiments, the acylated insulin is selected from the group consisting of the following insulins: A14E, B16H, B25H, B29K(N(ε)-docosanedioyl-yGlu-3xOEG), desB30 human insulin; A14E, B16H, B25H, B29K(N(ε)-docosanedioyl-γGlu-4xOEG), desB30 human insulin; A14E, B16E, B25H, B29K(N(ε)-docosanedioyl-γGlu-3xOEG), desB30 human insulin; and A14E, B16E, B25H, B29K (N(ε)-docosanedioyl-γGlu-4xOEG), desB30 human insulin.

In other embodiments, the acylated insulin is A14E, B16H, B25H, B29K (N(ε)-docosanedioyl-γGlu-3xOEG), desB30 human insulin, or A14E, B16H, B25H, B29K (N(ε))-docosanedioyl-γGlu-4xOEG), desB30 human insulin.

The second aspect of the present invention provides a pharmaceutical composition, comprising the acylated insulin described in the first aspect of the present invention and one or more pharmaceutically acceptable excipients.

In some embodiments, the pharmaceutical composition comprising at least about 1.5 moles of zinc ions/6 moles of the acylated insulin; preferably comprising at least about 2.2 moles of zinc ions/6 moles of the acylated insulin; preferably comprising about 2.2-12 moles of zinc ions/6 moles of the acylated insulin; preferably comprising about 2.3-10 moles of zinc ions/6 moles of the acylated insulin; more preferably comprising about 2.3-5.6 moles of zinc ions/6 moles of the acylated insulin; more preferably comprising about 2.3-4.8 moles of zinc ions/6 moles of insulin; more preferably comprising about 2.3-3.7 moles of zinc ions/6 moles of the acylated insulin; more preferably comprising about 2.3-3 moles of zinc ions/6 moles of the acylated insulin.

In some embodiments, the pharmaceutical composition has a pH of about 6.5-8.5; preferably a pH of about 6.8-8.2; preferably a pH of about 7.0-8.2; preferably a pH of about 7.2-7.6; more preferably a pH of about 7.4 or about 7.6.

In some embodiments, the pharmaceutical composition further comprises glycerol, phenol, m-cresol, NaCl, Na₂HPO₄, and/or citric acid; preferably, the pharmaceutical composition further comprises glycerol, phenol, and NaCl; preferably, the pharmaceutical composition further comprises glycerol, phenol, *m*-cresol, and NaCl; preferably, the pharmaceutical composition further comprises glycerol, phenol, NaCl and Na₂HPO₄ preferably, the pharmaceutical composition further comprises glycerol, phenol, NaCl and citric acid; more preferably, the pharmaceutical composition further comprises glycerol, phenol, *m*-cresol, NaCl and Na₂HPO₄; more preferably, the pharmaceutical composition further comprises glycerol, phenol, *m*-cresol, NaCl and citric acid.

In some embodiments, the content of the glycerol is no more than about 2.5% (w/w), preferably no more than about 2% (w/w), preferably about 0.3% to about 2% (w/), preferably about 0.5% to about 1.8% (w/w), preferably about 0.7% to about 1.8% (w/w), preferably about 1% to about 1.7% (w/w).

In some embodiments, the content of the phenol is about 15-80mM, preferably about 25-75mM, preferably about 30-70mM, preferably about 35-70mM, preferably about 45-70mM, preferably about 45-65mM; preferably about 45mM, about 46mM, about 47mM, about 48mM, about 49mM, about 50mM, about 51mM, about 52mM, about 53mM, about 54mM, about 55mM, about 56mM, about 57mM, about 58mM, about 59mM, about 60mM, about 61mM, about 62mM, about 63mM, about 64mM, or about 65mM.

In some embodiments, the content of the *m*-cresol is about 0-35mM, preferably about 0-19mM, preferably about 0-15mM, preferably about 0mM, about 1mM, about 2mM, about 3mM, about 4mM, about 5mM, about 6mM, about 7mM, about 8mM, about 9mM, about 10mM, about 11mM, about 12mM, about 13mM, about 14mM, or about 15mM.

In some embodiments, the content of the NaCl is about 0-150mM, preferably about 5-120mM, preferably about 10-120mM, preferably about 10-100mM, preferably about 10-75mM, preferably about 10-50mM, preferably about 10-30mM, preferably about 10-20mM.

In some embodiments, the content of the Na₂HPO₄ is about 0-75mM, preferably about 5-60mM, preferably about 5-50mM, preferably about 5-25mM, preferably about 5-10mM.

In some embodiments, the content of the citric acid is about 0-2mg/ml, preferably about 0.1-1.5mg/ml, preferably about 0.2-1mg/ml, preferably about 0.25-0.875mg/ml, preferably about 0.25-0.5mg/ml.

In some embodiments, the content of the acylated insulin is higher than about 0.6mM, preferably about 1.2-9.0mM, preferably about 1.2-8.4mM, preferably about 2.1-7.2mM, preferably about 2.1-6.0mM, preferably about 2.1-4.2mM, preferably about 2.1-3.6mM.

In some embodiments, the acylated insulin is selected from the group consisting of the following insulins: A14E, B16H, B25H, B29K (N(ε)-docosanedioyl-yGlu-3xOEG), desB30 human insulin; A14E, B16H, B25H, B29K (N(ε)-docosanedioyl-γGlu-4xOEG), desB30 human insulin; A14E, B16H, B25H, B29K (N(ε)-tricosanedioyl-γGlu-3xOEG), desB30 human insulin; A14E, B16H, B25H, B29K (N(ε)-tricosanedioyl-γGlu-4xOEG), desB30 human insulin; A14E, B16H, B25H, B29K (N(ε)-tetracosanedioyl-γGlu-3xOEG), desB30 human insulin; A14E, B16H, B25H, B29K (N(ε)-tetracosanedioyl-yGlu-4xOEG), desB30 human insulin; A14E, B16E, B25H, B29K (N(ε)-docosanedioyl-γGlu-3xOEG), desB30 human insulin; A14E, B16E, B25H, B29K (N(ε)-docosanedioyl-γGlu-4xOEG), desB30 human insulin; A14E, B16E, B25H, B29K (N(ε)-tricosanedioyl-γGlu-3xOEG), desB30 human insulin; A14E, B16E, B25H, B29K (N(ε)-tricosanedioyl-yGlu-4xOEG), desB30 human insulin; A14E, B16E, B25H, B29K (N(ε)-tetracosanedioyl-γGlu-3xOEG), desB30 human insulin; and A14E, B16E, B25H, B29K (N(ε)-tetracosanedioyl-γGlu-4xOEG), desB30 human insulin.

In some embodiments, the pharmaceutical composition comprises about 2.1-4.2mM (preferably about 2.1-3.5mM, preferably about 2.1-2.8mM, about 2.1mM) of acylated insulin, about 1% to about 2% (preferably about 1.5%-1.7%, more preferably about 1.7%) (weight/weight) of glycerol, about 15mM-65mM (preferably about 30mM-60mM, more preferably about 45mM-60mM, more preferably about 45mM-55mM) of phenol, about 1.5-7.0 (preferably about 2.2-5.6, preferably about 2.3-4.8, preferably about 2.3-3.7, preferably about 2.3-3, more preferably about 2.3) moles of zinc ions/6 moles of acylated insulin, about 10-120mM (preferably about 20-50mM, more preferably about 20mM) of sodium chloride, about 0-25mM (preferably about 0-15mM, preferably about 0-10mM, more preferably about 10mM) *m*-cresol, and has a pH of about 7.0-8.2 (preferably about 7.4); the acylated insulin is A14E, B16H, B25H, B29K (N(ε)-docosanedioyl-γGlu-3xOEG), desB30 human insulin; A14E, B16H, B25H, B29K (N(ε)-docosanedioyl-γGlu-4xOEG), desB30 human insulin; A14E, B16E, B25H, B29K (N(ε)-docosanedioyl-γGlu-3xOEG), desB30 human insulin; or A14E, B16E, B25H, B29K (N(ε)-docosanedioyl-yGlu-4xOEG), desB30 human insulin.

In some embodiments, the pharmaceutical composition comprises about 2.1-4.2mM (preferably about 2.1-3.5mM, preferably about 2.1-2.8mM, preferably about 2.1mM) of acylated insulin, about 1% to about 2% (preferably about 1.5%-1.7%, more preferably about 1.7%) (weight/weight) of glycerol, about 15mM-65mM (preferably about 30mM-60mM, more preferably about 45mM-60mM, more preferably about 45mM-55mM) of phenol, about 0-25mM (preferably about 0-15mM, preferably about 0-10mM, more preferably about 10mM) of *m*-cresol, about 10-120mM (preferably about 20-50mM, more preferably about 20mM) of NaCl, about 1.5-7.0 (preferably about 2.2-5.6, preferably about 2.3-4.8, preferably about 2.3-3.7, preferably about 2.3-3 , preferably about 2.3) moles of zinc ions/6moles of acylated insulin, about 0.1-1.5 mg/ml (preferably about 0.2-1mg/ml, more preferably about 0.5mg/ml) of citric acid, and has a pH of about 7.0-8.2 (preferably about 7.4), the acylated insulin is A14E, B16H, B25H, B29K (N(ε)-docosanedioyl-γGlu-3xOEG), desB30 human insulin; A14E, B16H, B25H, B29K (N(ε)-docosanedioyl-γGlu-4xOEG), desB30 human insulin; A14E, B16E, B25H, B29K (N(ε)-docosanedioyl-γGlu-3xOEG), desB30 human Insulin; or A14E, B16E, B25H, B29K (N(ε)-docosanedioyl-yGlu-4xOEG), desB30 human insulin.

In some embodiments, the pharmaceutical composition comprises about 2.1-4.2mM (preferably about 2.1-3.5mM, preferably about 2.1-2.8mM, preferably about 2.1mM) of acylated insulin, about 1% to about 2% (preferably about 1.5%-1.7%, more preferably about 1.7%) (weight/weight) of glycerol, about 15mM-65mM (preferably about 30mM-60mM, more preferably 45mM-60mM, more preferably about 45mM-55mM) of phenol, about 0-25mM (preferably about 0-15mM, preferably about 0-10mM, more preferably about 10mM) of *m*-cresol, about 10-120mM (preferably about 20-50mM, more preferably about 20mM) of NaCl, about 1.5-7.0 (preferably about 2.2-5.6, preferably about 2.3-4.8, preferably about 2.3-3.7, preferably about 2.3-3, more preferably about 2.3) moles of zinc ions/6 moles of acylated insulin, about 2-40mM (preferably about 5-10mM, more preferably about 5mM) of disodium hydrogen phosphate, and having a pH of about 7.0-8.2 (preferably about 7.4), the acylated insulin is A14E, B16H, B25H, B29K (N(ε)-docosanedioyl-γGlu-3xOEG), desB30 human insulin; A14E, B16H, B25H, B29K (N(ε)-docosanedioyl-γGlu-4xOEG), desB30 human insulin; A14E, B16E, B25H, B29K (N(ε)-docosanedioyl-γGlu-3xOEG), desB30 human insulin; or A14E, B16E, B25H, B29K (N(ε)-docosanedioyl-yGlu-4xOEG), desB30 human insulin.

In some embodiments, the pharmaceutical composition comprises about 2.1mM of acylated insulin, about 1.7% (w/w) of glycerol, about 45mM of phenol, about 2.3 moles of zinc ions/6 moles of acylated insulin, about 20mM of sodium chloride, about 10mM of *m*-cresol, and has a pH of about 7.4, the acylated insulin is A14E, B16H, B25H, B29K (N(ε)-docosanedioyl-yGlu-3xOEG), desB30 human insulin; A14E, B16H, B25H, B29K (N(ε)-docosanedioyl-γGlu-4xOEG), desB30 human insulin; A14E, B16E, B25H, B29K (N(ε)-docosanedioyl-γGlu-3xOEG), desB30 human insulin; or A14E, B16E, B25H, B29K (N(ε)-docosanedioyl-γGlu-4xOEG), desB30 human insulin.

In some embodiments, comprising about 2.1mM of acylated insulin, about 1.7% (weight/weight) of glycerol, about 45mM of phenol, about 10mM of *m*-cresol, about 20mM of NaCl, about 2.3 moles of zinc ions/6 moles of acylated insulin A14E, B16H, B25H, B29K (N(ε)-docosanedioyl-γGlu-3xOEG), desB30 human insulin; A14E, B16H, B25H, B29K (N(ε)-docosanedioyl-yGlu-4xOEG), desB30 human insulin; A14E, B16E, B25H, B29K (N(ε)-docosanedioyl-γGlu-3xOEG), desB30 human insulin; or A14E, B16E, B25H, B29K (N(ε)-docosanedioyl-γGlu-4xOEG), desB30 human insulin.

In some embodiments, comprising about 2.1mM of acylated insulin, about 1.7% (weight/weight) of glycerol, about 45mM of phenol, about 10mM of *m*-cresol, about 20mM of NaCl, about 2.3 moles of zinc ions/6 moles of acylated insulin, about 5mM of disodium hydrogen phosphate, and having a pH value of about 7.4, the acylated insulin is A14E, B16H, B25H, B29K (N(ε)-docosanedioyl-γGlu-3xOEG), desB30 human insulin; A14E, B16H, B25H, B29K (N(ε)-docosanedioyl-γGlu-4xOEG), desB30 human insulin; A14E, B16E, B25H, B29K (N(ε)-docosanedioyl-γGlu-3xOEG), desB30 human insulin; or A14E, B16E, B25H, B29K (N(ε)-docosanedioyl-γGlu-4xOEG), desB30 human insulin.

In some embodiments, the pharmaceutical composition further comprises an insulinotropic GLP-1 compound which is not only impaired, but also unexpectedly exhibits more excellent drug efficacy, chemical stability, physical stability, duration of action, half-life in *vivo,* compared to the monotherapy of acylated insulin and GLP-1 compound. In particular, the acylated insulin and GLP-1 compound in the pharmaceutical composition of the present invention have unexpected synergistic drug effects. For example, the synergistic hypoglycemic effect and the Hb1Ac reduction effect. Compared with other combination formulations of acylated insulin and long-acting GLP-1 compounds (such as the combination formulation of liraglutide and insulin degludec (trade name: Xultophy), the combination formulation of the present invention has unexpectedly better drug efficacy, duration of action, half-life in *vivo,* physical stability, chemical stability, etc. The combination formulation provided by the invention comprising the acylated insulin and GLP-1 compound can well realize long pharmacokinetics (hereinafter also referred to as PK) feature, making it possible to subcutaneous treatment of diabetic patients once a week, twice a week, or less frequency.

In one embodiment, the pharmaceutical composition further comprises an insulinotropic GLP-1 compound selected from the group consisting of the following insulinotropic GLP-1 compounds:
*N-*ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Gly8, Arg34]GLP-1-(7-37) peptide,
*N*-ε²⁶-(23-carboxytricosanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(21-carboxyheneicosanoylamino)-4(*S*)-carboxybutanoyl-[Gly8, Arg34]GLP-1-(7-37) peptide,
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl] [Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl] [Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl] [Arg34]GLP-1-(7-37) peptide,
*N*-ε²⁶-(23-carboxytricosanoylamino)-4(*S*)-carboxybutanoyl-[Arg34]GLP-1-(7-37) peptide,
*N*-ε²⁶-(19-carboxynonadecanoylamino)-4(*S*)-carboxybutanoyl-[Arg34]GLP-1-(7-37) peptide,
*N*-ε²⁶-(21-carboxyheneicosanoylamino)-4(*S*)-carboxybutanoyl-[Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Gly8, Arg34]GLP-1-(7-37) peptide,
*N*-ε²⁶-(20-carboxyeicosanoylamino)-4(*S*)-carboxybutanoyl-[Gly8, Arg34]GLP-1-(7-37) peptide,
*N*-ε²⁶-(22-carboxydocosanoylamino)-4(*S*)-carboxybutanoyl-[Gly8, Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl] [Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl
][Arg34]GLP-1-(7-37) peptide, N-ε²⁶-[2-(2-[2-(4-[22-carboxydocosanoylamino]-4(*S*)-carboxybutanoylamino)ethoxy]ethoxy)acetyl] [Arg34]GLP-1-(7-37) peptide,
*N*-ε²⁶-(20-carboxyeicosanoylamino)-4(*S*)-carboxybutanoyl-[Arg34]GLP-
1-(7-37) peptide, *N*-ε²⁶-(22-carboxydocosanoylamino)-4(*S*)-carboxybutanoyl-[Arg34]GLP-1-(7-37) peptide,
*N*-ε²⁶-(17-carboxyheptadecanoylamino)-4(*S*)-carboxybutanoyl-Arg34]GLP-1-(7-37) peptide,
*N*-ε²⁶-(17-carboxyheptadecanoylamino)-4(*S*)-carboxybutanoyl-[Gly8,
Arg34]GLP-1-(7-37) peptide,
*N*-ε²⁶-[2-(2-[2-(2-[2-(2-[4-(17-carboxyheptadecanoylamino)-4(*S*)-carboxybutanoylamino]ethoxy)ethoxy]acetylamino)ethoxy]ethoxy)acetyl ][Aib8, Arg34]GLP-1-(7-37) peptide,
and *N*-ε²⁶-[2-(2-[2-(2-[2-(2-[4-(17-carboxyheptadecanoylamino)-4(*S*)-carboxybutanoylamino]ethoxy)ethoxy]acetylamino)ethoxy]ethoxy)acetyl ][Gly8, Arg34]GLP-1-(7-37) peptide.

In some embodiments, the insulinotropic GLP-1 compound is selected from the group consisting of:
*N*-ε²⁶-2-(2-[2-(2-[2-(2-[4-(19-carboxynonadecanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl-[Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl ][Gly8, Arg34]GLP-1-(7-37) peptide, and
*N*-ε²⁶-[2-(2-[2-(4-[21-carboxyheneicosanoylamino]-4(*S*)-carboxybutanoylamino)ethoxy]ethoxy)acetyl][Gly8, Arg34]GLP-1-(7-37) peptide.

Preferably, the insulinotropic GLP-1 compound is:
*N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide, or *N*-ε²⁶-[2-(2-[2-(2-[2-(2-[4-(21-carboxyheneicosanoylamino)-4(S)-
carboxybutanoylamino]ethoxy)ethoxy]acetylamino)ethoxy]ethoxy)acetyl ][Gly8, Arg34]GLP-1-(7-37) peptide.

In some embodiments, the molar ratio of the insulinotropic GLP-1 compound to the acylated insulin is at least about 1:100, preferably at least about 3:100, preferably at least about 5:100, preferably at least about 8:100, preferably about (3:100)-(100:100), preferably about (5:100)-(80:100), preferably about (8:100)-(50:100), preferably about (10:100)-(50:100), preferably about (13:100)-(50:100), preferably about (13:100)-(40:100), preferably about (13:100) 100)-(35:100), preferably about (13:100)-(27:100), preferably about (13:100)-(20:100).

The inventors unexpectedly found that when the molar ratio of the insulinotropic GLP-1 compound to the acylated insulin in the pharmaceutical composition of the second aspect of the present invention is a specific ratio, it can achieve better efficacy than the single formulation comprising double content of the acylated insulin and the single formulation comprising double content of the GLP-1 compound.

The third aspect of the present invention provides the acylated insulin according to the first aspect of the present invention or the pharmaceutical composition according to the second aspect for use as a medicine. In some embodiments, the acylated insulin or the pharmaceutical composition is used as a medicament for treating diabetes.

The fourth aspect of the present invention provides use of the acylated insulin described in the first aspect of the present invention or the pharmaceutical composition described in the second aspect in the manufacture of medicament for treating diabetes.

The fifth aspect of the present invention provides a method for treating diabetes, comprising administering a therapeutically effective amount of the acylated insulin described in the first aspect of the present invention or the pharmaceutical composition described in the second aspect to a subject in need thereof.

The sixth aspect of the present invention provides a method for treating diabetes, comprising administering a therapeutically effective amount of an acylated insulin of formula (A), or a pharmaceutically acceptable salt, amide or ester thereof, to a subject in need, wherein the acylated insulin is administered to the subject every 4 days or less frequency:
III-(II)ₘ-(I)ₙ-ins (A),
wherein, ins is the insulin parent of the acylated insulin, III-(II)ₘ-(I)ₙ is the acyl moiety of the acylated insulin, the insulin parent is A14E, B16H, B25H, desB30 human insulin or A14E, B16E, B25H, desB30 human insulin, the acyl moiety is linked to the ε amino group of the lysine residue at position B29 of the insulin parent, I is a neutral, alkylene glycol-containing amino acid residue, II is an acidic amino acid residue, III is a fatty diacid comprising 22, 23, 24, 25, or 26 carbon atoms, wherein formally, a hydroxyl group has been removed from one of the carboxyl groups in the fatty diacid, III, II, and I are linked by amide bonds, and the order of II and I presented in formula (A) can be interchanged independently; m is 1, 2, 3, 4 or 5, and n is an integer of 3 or 4.

In some embodiments, the acylated insulin is administered to the subject every 5 days or less frequency; preferably, the acylated insulin is administered to the subject every 6 days or less frequency; Preferably, the acylated insulin is administered to the subject every 7 days or less frequency; preferably, the acylated insulin is administered to the subject every 8 days or less frequency; preferably, the acylated insulin is administered to the subject every 9 days or less frequency; preferably, the acylated insulin is administered to the subject every 10 days or less frequency; preferably, the subject is administered every 2 weeks or more frequency.

In some embodiments, I is: -HN-(CH₂)₂-O-(CH₂)₂-O-CH₂-CO-, -HN-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₃-O-(CH₂)₄-O-(CH₂)₃-NH-CO-, -HN-(CH₂)₃-O-(CH₂)₄-O-(CH₂)₃-NH-CO-CH₂-O-CH₂-CO-, -HN-(CH₂)₃-O-(CH₂)₄-O-(CH₂)₃-NH-CO-(CH₂)₂-CO-, -HN-(CH₂)₂-O-(CH₂)₂-O-CH₂-CO-CH₂-O-CH₂-CO-, -HN-(CH₂)₃-O-(CH₂)₂-O-(CH₂)₂-O-(CH₂)₃-NH-CO-(CH₂)₂-CO-, -HN-(CH₂)₃-O-(CH₂)₂-O-(CH₂)₂-O-(CH₂)₃-NH-CO-CH₂-O-CH₂-CO-, -HN-(CH₂)₂-O-(CH₂)₂-O-(CH₂)₂-NH-CO-(CH₂)₂-CO-, - HN-(CH₂)₂-O-(CH₂)₂-O-(CH₂)₂-NH-CO-CH₂-O-CH₂-CO-, -HN-(CH₂)₃-O-(CH₂)₂-O-(CH₂)₂-O-(CH₂)₃-NH-CO-CH₂-O-CH₂-CO-, -HN-(CH₂)₃-O-(CH₂)₃-O-CH₂-CO-, or -HN-(CH₂)₄-O-(CH₂)₄-O-CH₂-CO-; preferably I is -HN-(CH₂)₂-O-(CH₂)₂-O-CH₂-CO-.

In some embodiments, II is an amino acid residue selected from the group consisting of γGlu, αGlu, βAsp, αAsp, γ-D-Glu, α-D-Glu, β-D-Asp and αD-Asp, preferably, II is γGlu.

In some embodiments, III is a fatty diacid comprising 22, 23 or 24 carbon atoms, wherein formally a hydroxyl group has been removed from one of the carboxyl groups of the fatty diacid, preferably III is HOOC-(CH₂)₂₀-CO-, HOOC-(CH₂)₂₁-CO-, or HOOC-(CH₂)₂₂-CO-, preferably, III is HOOC-(CH₂)₂₀-CO-.

In some embodiments, n is 3. In some embodiments, m is 1 or 2.

In some embodiments, the acylated insulin is selected from the group consisting of the following insulins: A14E, B16H, B25H, B29K (N(ε)-docosanedioyl-yGlu-3xOEG), desB30 human insulin; A14E, B16H, B25H, B29K (N(ε)-docosanedioyl-γGlu-4xOEG), desB30 human insulin; A14E, B16H, B25H, B29K (N(ε)-tricosanedioyl-γGlu-3xOEG), desB30 human insulin; A14E, B16H, B25H, B29K (N(ε)-tricosanedioyl-γGlu-4xOEG), desB30 human insulin; A14E, B16H, B25H, B29K (N(ε)-tetracosanedioyl-yGlu-3xOEG), desB30 human insulin; A14E, B16H, B25H, B29K (N(ε)-tetracosanedioyl-yGlu-4xOEG), desB30 human insulin; A14E, B16E, B25H, B29K (N(ε)-docosanedioyl-γGlu-3xOEG), desB30 human insulin; A14E, B16E, B25H, B29K (N(ε)-docosanedioyl-γGlu-4xOEG), desB30 human insulin; A14E, B16E, B25H, B29K (N(ε)- tricosanedioyl-yGlu-3xOEG), desB30 human insulin; A14E, B16E, B25H, B29K (N(ε)-tricosanedioyl-yGlu-4xOEG), desB30 human insulin; A14E, B16E, B25H, B29K (N(ε)-tetracosanedioyl-γGlu-3xOEG), desB30 human insulin; and A14E, B16E, B25H, B29K (N(ε)-tetracosanedioyl-γGlu-4xOEG), desB30 human insulin. Preferably, the acylated insulin is selected from the group consisting of the following insulins: A14E, B16H, B25H, B29K (N(ε)-docosanedioyl-yGlu-3xOEG), desB30 human insulin; A14E, B16H, B25H, B29K (N(ε)-docosanedioyl-γGlu-4xOEG), desB30 human insulin; A14E, B16E, B25H, B29K (N(ε)-docosanedioyl-γGlu-3xOEG), desB30 human insulin; and A14E, B16E, B25H, B29K (N(ε)-docosanedioyl-γGlu-4xOEG), desB30 human insulin; more preferably, the acylated insulin is A14E, B16H, B25H, B29K (N(ε)-docosanedioyl-γGlu-3xOEG), desB30 human insulin, or A14E, B16H, B25H, B29K (N(ε))-docosanedioyl-γGlu-4xOEG), desB30 human insulin.

In some embodiments, the diabetes is type 1 diabetes or type 2 diabetes.

### BRIEF DESCRIPTION OF THE DRAWINGS

FIG. 1a shows the hypoglycemic effect of compound 1 of the present invention, control compound 2 and vehicle on streptozotocin (STZ)-induced type I diabetic (T1DM) rats.
FIG. 1b shows, in corresponding to FIG. 1a, the ΔAUC of the hypoglycemic effect of compound 1 of the present invention, control compound 2, and vehicle on STZ-induced T1DM rats.
FIG. 1c shows the HbA1c-lowering effect of compound 1 of the present invention, control compound 2 and vehicle on STZ-induced T1DM rats.
FIG. 1d shows the hypoglycemic effect of compound 1 of the present invention, control compound 2 and vehicle on streptozotocin (STZ)-induced type I diabetic (T1DM) rats.
FIG. 1e shows, in corresponding to FIG. 1d, the ΔAUC of the hypoglycemic effect of compound 1 of the present invention, control compound 2, and vehicle on STZ-induced T1DM rats.
FIG. 2a shows the hypoglycemic effect of compound 1 of the present invention, control compound 2 and vehicle on db/db mice.
FIG. 2b shows, in corresponding to FIG. 2a, the ΔAUC of the hypoglycemic effect of compound 1 of the present invention, control compound 2, and vehicle on db/db mice.
FIG. 3a shows the hypoglycemic effect of compound 1 of the present invention, control compound 2, and vehicle on db/db mice in an oral glucose tolerance experiment.
FIG. 3b shows, in corresponding to FIG. 3a, the AUC of the glucose-lowering effect of compound 1 of the present invention, control compound 2, and vehicle on db/db mice in an oral glucose tolerance experiment.
FIG. 4a shows the hypoglycemic effect of compound 1 of the present invention, control compound 2 and vehicle on streptozotocin (STZ)-induced type I diabetic (T1DM) rats.
FIG. 4b shows, in corresponding to FIG. 4a, the ΔAUC of the hypoglycemic effect of compound 1 of the present invention, control compound 2, and vehicle on STZ-induced T1DM rats.
FIG. 4c shows the HbA1c lowering effect of compound 1 of the present invention, control compound 2 and vehicle on STZ-induced type I diabetic (T1DM) rats.
FIG. 5a shows the hypoglycemic effect and duration of action of the compounds of Comparative Examples 3-4 of the present invention and vehicle on db/db mice.
FIG. 5b shows, in correspondence with FIG. 5a, the AUC of the hypoglycemic effect of the compounds of Comparative Examples 3-4 of the present invention and vehicle on db/db mice.

### DETAILED DESCRIPTION

### Definitions

Herein, the term "insulin" comprises natural insulins, such as human insulin, the insulin analogues and the insulin derivatives thereof.

The term "insulin analogue" comprises a polypeptide having a molecular structure which may be formally derived from the structure of a natural insulin (e.g., human insulin) by deletion and/or substitution (replacement) of one or more amino acid residues presented in the natural insulin and/or by addition of one or more amino acid residues. The amino acid residues for addition and/or substitution may be encodable amino acid residues, or other natural amino acid residues, or purely synthetic amino acid residues. Preferably, the amino acid residues for addition and/or substitution are encodable amino acid residues.

Herein, the term "insulin derivative" refers to a natural insulin or insulin analogue which has been chemically modified, and the modification may be, for example, introducing a side chain at one or more positions of the insulin backbone, oxidizing or reducing groups of amino acid residues on the insulin, converting a free carboxyl group into an ester group, or acylating a free amino group or a hydroxyl group. The acylated insulins of the present invention are insulin derivatives.

The term "insulin parent" refers to an insulin moiety of an insulin derivative or an acylated insulin (also referred to herein as insulin parent), and, for example, it refers to a moiety of an insulin derivative or an acylated insulin without a linking side chain or an added acyl group in the present invention. The insulin parent may be a natural insulin, such as human insulin or porcine insulin. In another aspect, the insulin parent may be an insulin analogue.

Herein, the term "amino acid residue" comprises amino acids from which a hydrogen atom has been removed from an amino group and/or a hydroxyl group has been removed from a carboxyl group and/or a hydrogen atom has been removed from a mercapto group. Imprecisely, an amino acid residue may be referred to as an amino acid.

Herein, acidic amino acid residue comprises an amino acid having a molecular weight of up to about 200 Da, comprising two carboxylic acid groups and a primary or secondary amino group. Alternatively, acidic amino acid residue is an amino acid having a molecular weight of up to about 250 Da and comprising a carboxylic acid group and a primary or secondary sulfonamide group.

Unless otherwise stated, all amino acids referred to herein are L-amino acids.

The insulin derivatives or acylated insulins, analogs and intermediates in the invention may be in the form of pharmaceutically acceptable salts, amides or esters. The salt may be a base salt, an acid salt, or a neutral salt. In water, alkaline salts produce hydroxide ions and acid salts produce hydrated hydrogen ions. Salts of the derivatives herein may be formed with added cations or anions that react with an anionic group or a cationic group, respectively. These groups may be located within the peptide portion and/or within the side chain of the insulin derivative or acylated insulin of the present invention.

Non-limiting examples of the anionic moieties of the acylated insulin of the present invention comprise side chains and free carboxyl groups in the peptide portion. The peptide portion typically comprises a free carboxylic acid at the C-terminus, and it may also include free carboxyl groups on internal acidic amino acid residues such as Asp and Glu.

Non-limiting examples of cationic groups in the peptide portion include free amino groups at the N-terminus, if any, and any free amino groups on internal basic amino acid residues such as His, Arg and Lys.

Esters of the acylated insulin or insulin derivatives of the present invention may be formed, for example, by the reaction of a free carboxylic acid group with an alcohol or phenol, which results in the substitution of at least one hydroxyl group by an alkoxy or aryloxy group. The formation of the ester may involve a free carboxyl group at the C-terminus of the peptide, and/or any free carboxyl group of the side chain.

The amide of the acylated insulin or insulin derivative of the present invention may be generated, for example, by reaction of a free carboxylic acid group with an amine or substituted amine, or by reaction of a free or substituted amino group with a carboxylic acid. The formation of the amide may involve a free carboxyl group at the C-terminus of the peptide, any free carboxyl group in the side chain, a free amino group at the N-terminus of the peptide, and/or any free or substituted peptide amino group in the peptide and/or side chain.

In one specific example, the acylated insulin or insulin derivative of the present invention is in the form of a pharmaceutically acceptable salt. In another specific example, in the form of a pharmaceutically acceptable amide, preferably having an amide group at the C-terminus of the peptide. In a further specific example, the peptide or derivative is in the form of a pharmaceutically acceptable ester.

Herein, the term "alkylene glycol" comprises oligo- and poly-alkylene glycol moieties and monoalkylene glycol moieties. Monoalkylene glycols and polyalkylene glycols include, for example, chains based on monoethylene and polyethylene glycols, monopropylene and polypropylene glycols, and monotetramethylene and polytetramethylene glycols, i.e., chains based on the repeating unit -CH₂CH₂O-, - CH₂CH₂CH₂O- or -CH₂CH₂CH₂CH₂O-. The alkylene glycol moiety can be monodisperse (with well-defined length/molecular weight) and polydisperse (with less well-defined length/average molecular weight). The monoalkylene glycol moiety includes -OCH₂CH₂O-, - OCH₂CH₂CH₂O- or -OCH₂CH₂CH₂CH₂O- comprising different groups at each end.

Herein, the term "fatty diacid" includes linear or branched fatty dicarboxylic acids having at least two carbon atoms and being saturated or unsaturated. Non-limiting examples of fatty diacids are hexanedioic acid, octanedioic acid, decanedioic acid, dodecanedioic acid, tetradecanedioic acid, hexadecanedioic acid, heptadecanedioic acid, octadecanedioic acid, eicosanedioic acid, docosanedioic acid and tetracosanedioic acid.

As used herein, rapid-acting insulins include rapid-acting natural insulins, insulin analogues and insulin derivatives. Rapid-acting insulin typically begins to act within, for example, 1 to 20 minutes, peaks after about one hour, and continues to act for three to five hours.

The term "basal insulin" refers to an insulin having a longer duration of action than conventional or normal human insulin.

Herein, the term "chemical stability" means that the insulin derivatives disclosed in the present invention are chemically sufficiently stable in a desired formulation. That is, chemical degradation products are formed in just an amount that does not impair the shelf life of the final drug product. Chemical degradation products include deamidation products, products from the formation of isoaspartate ester, the formation of dimer, the racemization, the dehydration process and the like. Chemical stability can be determined by HPLC analysis of aged samples or formulations.

High physical stability means that the fibrillation tendency is less than 50% of that of human insulin. Fibrillation can be described by the lag time before fibrillation starts to form under given conditions.

Polypeptides having affinity for an insulin receptor and an IGF-1 receptor are polypeptides that are capable of interacting with the insulin receptor and the human IGF-1 receptor in a suitable binding assay. Such receptor assays are well known in the art.

As used herein, "drug effect" or "potency" refers to the ability of a drug or an active compound to result in a certain function or effect (e.g., lowering blood glucose). For example, compared with insulin degludec or other existing insulin derivatives, administration of the same dose of an insulin derivative of the present invention will result in a better blood glucose lowering effect or function.

The term "diabetes" includes type 1 diabetes, type 2 diabetes, gestational diabetes (during pregnancy) and other conditions that cause hyperglycemia. The term is used for metabolic disorders in which the pancreas produces insufficient amount of insulin or in which cells of the body fail to respond appropriately to insulin, thereby preventing the cells from taking up glucose. As a result, glucose accumulates in the blood. Type 1 diabetes, also known as insulin-dependent diabetes mellitus (IDDM) and juvenile onset diabetes, is caused by β -cell destruction and often results in absolute insulin deficiency. Type 2 diabetes, also known as non-insulin dependent diabetes mellitus (NIDDM) and maturity-onset diabetes, is associated with major insulin resistance and thus major defects in insulin secretion featuring relative insulin deficiency and/or insulin resistance.

As used herein, the term "GLP-1 analogue" or "analogue of GLP-1" refers to a peptide or compound that is a variant of human glucagon-like peptide-1 (GLP-1(7-37)), wherein one or more amino acid residues of GLP-1(7-37) are replaced, and/or one or more amino acid residues are deleted, and/or one or more amino acid residues are added. Specifically, the sequence of GLP-1(7-37) is shown in SEQ ID NO: 15 in the sequence listing. A peptide having the sequence shown in SEQ ID NO: 15 may also be referred to as "natural" GLP-1 or "natural" GLP-1(7-37).

In the sequence listing, the first amino acid residue (His) in SEQ ID NO: 5 is numbered 1. However, in the following, according to established practice in the art, the histidine residue is numbered 7 and the following amino acid residues are numbered sequentially, ending with glycine as No. 37. Thus, in general, based on the numbering for amino acid residues or positions, the GLP-1(7-37) sequence referred to herein is a sequence starting with His at position 7 and ending with Gly at position 37.

[Gly8, Arg34]GLP-1-(7-37) peptide is a GLP-1 analogue having Gly and Arg at positions corresponding to position 8 and position 34, respectively, of GLP-1(7-37) (SEQ ID NO: 5). [Arg34]GLP-1-(7-37) peptide is a GLP-1 analogue having Arg at a position corresponding to position 34 of GLP-1(7-37) (SEQ ID NO: 5). Specifically, the amino acid sequences of [Gly8, Arg34]GLP-1-(7-37) peptide and [Arg34]GLP-1-(7-37) peptide are shown in SEQ ID NO: 6 and SEQ ID NO: 7 in the sequence listing, respectively. In the case of a GLP-1 peptide or an analogue thereof, the term "derivative" as used herein refers to a chemically modified GLP-1 peptide or analogue, wherein one or more substituents have been covalently linked to the peptide. Substituents may also be referred to as side chains.

As used herein, the naming of insulin or GLP-1 compounds follows the following principles: the names are given according to mutations and modifications (e.g., acylation) relative to human insulin, or mutations and modifications (e.g., acylation) relative to natural GLP-1(7-37). The naming of the acyl moieties is based on the IUPAC nomenclature and, in other cases, the peptide nomenclature. For example, name the following acyl moiety: 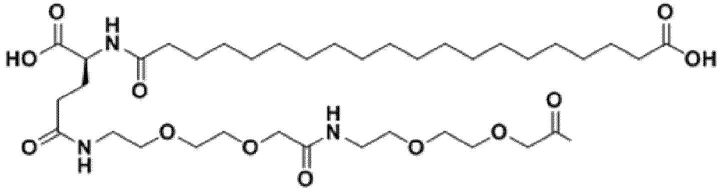 for example, it can be named as "eicosanedioyl-yGlu-OEG-OEG", "eicosanedioyl-γGlu-2×OEG" or "eicosanedioyl-gGlu-2×OEG", or "19-carboxynonadecanoyl-yGlu-OEG-OEG", wherein OEG is the shorthand for the group -NH(CH₂)₂O(CH₂)₂OCH₂CO- (i.e., 2-[2-(2-aminoethoxy)ethoxy]acetyl) and γGlu (or gGlu) is a shorthand for the amino acid γ-glutamic acid in the L configuration. Alternatively, the acyl moieties may be named according to IUPAC nomenclature (OpenEye, IUPAC format). According to this nomenclature, the above acyl moiety of the present invention is referred to as the following name: [2-(2-[2-(2-[2-(2-[4-(19-carboxynonadecanoylamino)-4(S)-carboxybutanoylamino]ethoxy)ethoxy]acetylamino)ethoxy]ethoxy)acetyl ], or [2-[2-[2-[2-[2-[2-[(4S)-4-carboxy-4-(19-carboxynonadecanoylamino)butanoyl]-amino]-ethoxy]-ethoxy]acetyl]aminoethoxy]ethoxy]acetyl].

For example, the insulin having the sequence/structure given below is referred to as "B29K(N(ε)-docosanedioyl-γGlu-3×OEG), desB30 human insulin","B29K(N^{ε}-docosanedioyl-yGlu-3×OEG), desB30 human insulin", or "B29K(N^{ε}-docosanedioyl-gGlu-3×OEG), desB30 human insulin", which indicates that the amino acid K at position B29 in human insulin has been modified by acylation with the residue eicosanedioyl-gGlu-3×OEG on the ε nitrogen (referred to as N^{ε} or (N(ε)) of the lysine residue at position B29, and the amino acid T at position B30 in human insulin has been deleted. For another example, the insulin having the sequence/structure given below is referred to as "A14E, B16H, B25H, B29K(Nε-docosanedioyl-gGlu-3×OEG), desB30 human insulin" or "A14E, B16H, B25H, B29K(N(ε)-docosanedioyl-γGlu-3×OEG), desB30 human insulin", which indicates that amino acid Y at position A14 in human insulin has been mutated to E, amino acid Y at position B16 in human insulin has been mutated to H, amino acid F at position B25 in human insulin has been mutated to H, amino acid K at position B29 in human insulin has been modified by acylation with the residue docosanedioyl-gGlu-3×OEG on the ε nitrogen (referred to as N^{ε}) of the lysine residue at position B29, and amino acid T at position B30 in human insulin has been deleted.

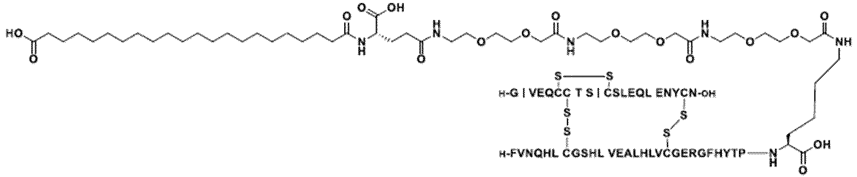

Insulin is a polypeptide hormone secreted by β cells in the pancreas and is composed of two polypeptide chains, namely A chain and B chain, linked by two inter-chain disulfide bonds. In addition, the A chain is characterized by having an intra-chain disulfide bond.

There are three main methods for preparing human insulin in microorganisms. Two of those methods involve *E. coli,* one by expressing fusion proteins in the cytoplasm (Frank et al. (1981) in Peptides: Proceedings of the 7th American Peptide Chemistry Symposium (Rich & Gross, eds.), Pierce Chemical Co., Rockford, III, pp. 729-739), and the other by using signal peptides to enable the secretion of it into the periplasmic space (Chan et al. (1981) PNAS 78:5401-5404). The third method involves enabling the secretion of an insulin precursor into the medium by means of *Saccharomyces cerevisiae* (Thim et al. (1986) PNAS 83:6766-6770). A number of methods for the expression of insulin precursors in *E. coli* or *Saccharomyces cerevisiae* have been disclosed in the prior art. See, e.g., U.S. Patent No. 5,962,267, WO95/16708, EP0055945, EP0163529, EP0347845 and EP0741188.

Construction of a vector, expression, processing and purification of an insulin analogue can be carried out using techniques well known to those skilled in the art. For example, the insulin analogue can be prepared by expressing a DNA sequence encoding the target insulin analogue in a suitable host cell by the well-known techniques disclosed in U.S. Patent No. 6500645. For example, insulin analogues can also be prepared by methods reported in the following paper: Glendorf T, Sorensen AR, Nishimura E, Pettersson I, & Kjeldsen T: Importance of the Solvent-Exposed Residues of the Insulin B Chain α-Helix for Receptor Binding; Biochemistry, 2008, 47:4743-4751. In this paper, mutations are introduced into an insulin-encoding vector using overlap extension PCR. Insulin analogues are expressed in *Saccharomyces cerevisiae* strain MT663 as proinsulin-like fusion proteins with an Ala-Ala-Lys mini C-peptide. The single-chain precursors are enzymatically converted into two-chain desB30 analogues by using a hydrolyzed Achromobacter lyticus (*A. lyticus)* endoprotease.

Isolated insulin analogues can be acylated at the desired position by acylation methods well known in the art, and examples of such insulin analogues are described in, for example, Chinese Patent Application Publication Nos. CN1029977C, CN1043719A and CN1148984A. Nucleic acid sequences encoding polypeptides of the insulin analogues can be prepared synthetically by established standard methods, for example, by the method described in Beaucage et al. (1981) Tetrahedron Letters 22:1859-1869 or Matthes et al. (1984) EMBO Journal 3:801-805.

The term "excipient" broadly refers to any component other than the active therapeutic ingredient. The excipient may be inert substances, inactive substances and/or non-pharmaceutically active substances.

The excipient may be used for various purposes, depending on the pharmaceutical composition, for example as carriers, vehicles, diluents, tablet aids, and/or for improving administration and/or absorption of the active substances. Examples of excipients include, but are not limited to, diluents, buffers, preservatives, tension regulators (also known as tonicity agents or isotonic agents), chelating agents, surfactants, protease inhibitors, wetting agents, emulsifiers, antioxidants, fillers, metal ions, oily vehicles, proteins, and/or zwitterions, and stabilizers.

Pharmaceutical compositions of pharmaceutically active ingredients with various excipients are known in the art, see, e.g., Remington: The Science and Practice of Pharmacy (e.g., 19th edition (1995), and any later versions). For the convenience of the patient, it is assumed that the time interval (time delays) from the administration of the acylated insulin of the present invention to the next administration of the acylated insulin of the present invention by the patient is of the same length or approximately the same length in days. Patient will administer the acylated insulin of the present invention once a week, i.e. on the same day of a week, e.g., every Sunday. This would be that the acylated insulin is administered, on average over a period of 1 month, 6 months or 1 year, every 6 days and not at a higher frequency.

Diseases and conditions that are the primary targets of the present invention are diabetes (type 1 or type 2) or other conditions characterized by hyperglycemia, but mostly metabolic diseases and conditions in which the metabolic action of insulin has clinical relevance or benefits, such as pre-diabetes, impaired glucose tolerance, metabolic syndrome, obesity, cachexia, *in vivo* β-cell damage/death, bulimia and inflammation. All of these types of conditions are known or believed to benefit from a stable metabolic state in a subject suffering from the disease or condition. In any event, any treatment regimen which comprises the administration of insulin can be varied by practicing the teachings of the present invention; that is, such therapy will comprise the administration of insulin with prolonged duration of action provided herein.

### Examples

The following examples are provided by way of illustration but not limitation.

Abbreviations used herein are as follows:
OEG is the amino acid residue -NH(CH₂)₂O(CH₂)₂OCH₂CO-;
OSu is succinimidyl-1-yloxy-2,5-dioxo-pyrrolidin-1-yloxy;
OtBu is oxy-*tert*-butyl;
HCl is hydrogen chloride;
γGlu or gGlu is yL-glutamoyl;
NHS is *N*-hydroxysuccinimide;
DCC is dicyclohexylcarbodiimide;
AEEA is 2-(2-(2-aminoethoxy)ethoxy)acetic acid;
OH is hydroxyl;
CH₃CN is acetonitrile;
Gly is glycine;
Arg is arginine;
TFA is trifluoroacetic acid;
HbA1c is glycated hemoglobin;
AUC is the area under the curve of the time-blood glucose curve;

The following examples and general methods are directed to intermediate compounds and final products determined in the specification and synthetic schemes. The preparation of the compounds of the present invention is described in detail using the following examples, but the chemical reactions described are disclosed in terms of their general applicability to the preparation of the compounds of present the invention. Sometimes, the reaction may not be applicable to every compound within the scope of the present invention as described. Those skilled in the art will readily recognize compounds for which this will occur. In these cases, the reaction can be successfully carried out by conventional modifications known to those skilled in the art, that is, by suitable protection of interfering groups, by change into other conventional reagents, or by conventional modifications of the reaction conditions. In all preparation methods, all starting materials are known or can be readily prepared using known starting materials. All temperatures are given in degrees celsius and, unless otherwise explicitly stated; all parts and percentages are by weight when referring to yield, and all parts are by volume when referring to a solvent and an eluent.

### Examples

### Example 1

A14E, B16H, B25H, B29K(N(ε)-docosanedioyl-γGlu-3xOEG), desB30 human insulin (compound 1)

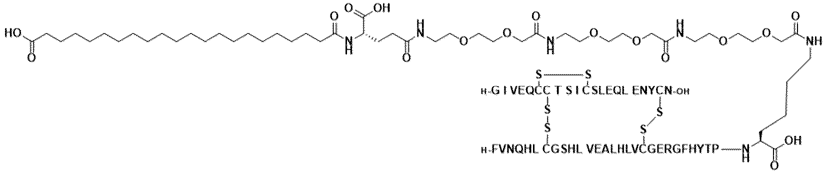

### 1, Preparation of A14E, B16H, B25H, B29K(N(ε)-docosanedioyl-γGlu-3xOEG), desB30 human insulin

A14E, B16H, B25H, desB30 human insulin was prepared by conventional methods of preparing insulin analogs (see Glendorf T, Sorensen AR, Nishimura E, Pettersson I, & Kjeldsen T: Importance of the Solvent-Exposed Residues of the Insulin B Chain α-Helix for Receptor Binding; Biochemistry 2008 47 4743-4751). A14E, B16H, B25H, desB30 human insulin (10g, 1.78 mmol) was dissolved in 100mM aqueous Na₂HPO₄ solution (370mL) and acetonitrile (370mL) was added. The pH was adjusted to 11.0-11.4 with 1N NaOH. *tert*-Butyl docosanedioyl-yGlu-(3xOEG-OSu)-OtBu (2.24g, 1.96 mmol) was dissolved in acetonitrile (20 mL), and the solution was slowly added to the insulin solution. The pH was maintained at 11.0-11.4. After 30min, the reaction mixture was added to water (370mL), and the pH was adjusted to 7.8 with 1N NC1 solution. The precipitate was separated out by centrifugation and lyophilized. The crude product was added to a mixed solution of trifluoroacetic acid (100mL) and dichloromethane (100mL), and the mixture was stirred at room temperature for 60 min. The mixture was then concentrated to about 50 mL and poured into ice-cold n-heptane (500mL), and the precipitated product was isolated by filtration and washed twice with n-heptane. The resulting precipitate was dried under vacuum and purified by ion exchange chromatography (Resource Q, 0.25%-1.25% ammonium acetate gradient in 42.5% ethanol, pH 7.5) and reverse phase chromatography (acetonitrile, water, TFA). The purified fractions were combined, adjusted to pH 5.2 with 1 N HCl, and separated to obtain the precipitate, which was lyophilized to obtain the compound 1. LC-MS (ESI): m/z = 1092.5367[M+6H]⁶⁺

### 2, Preparation of intermediate tert-butyl docosanedioyl-γGlu-(3xOEG-OSu)-OtBu

### 2.1 Tert-butyl docosanedioyl-OSu

Docosanedioic acid mono-*tert*-butyl ester (20 g, 46.87mMo1) and NHS (5.39 g, 46.87mMo1) were mixed in dichloromethane under nitrogen atmosphere, and triethylamine (20mL) was added. The resulting turbid mixture was stirred at room temperature, added with DCC (10.64g, 51.56mMo1) and further stirred overnight. The reaction mixture was filtered, and the resulting filtrate was concentrated to almost dry. The residue was mixed with cold water and ethyl acetate, and the mixture was stirred for 20 min and subjected to liquid separation. The upper organic phase was washed with saturated brine, and after liquid separation, the upper organic phase was dried over anhydrous sodium sulfate and filtered. The filtrate was concentrated to almost dry under reduced pressure and dried overnight under vacuum to obtain *tert*-butyl docosanedioyl-OSu (23.32 g, yield 95%).

### LC-MS (Scie×100API): m/z = 523.39(M+1)⁺

### 2.2 Tert-butyl docosanedioyl-yGlu-OtBu

*Tert*-butyl docosanedioyl-OSu (23.32g, 44.52mmol) was dissolved in dichloromethane (230mL), and the solution was stirred and added with H-Glu-OtBu (9.95g, 48.97mmol), triethylamine (19mL) and water sequentially. The mixture was heated to obtain a clarified solution, which was then stirred at room temperature for 4h. Then, the reaction solution was added with 10% aqueous citric acid solution (230mL) and subjected to liquid separation. The lower organic phase was washed with saturated brine, and after liquid separation, the lower organic phase was dried over anhydrous sodium sulfate and filtered. The filtrate was concentrated to almost dry under reduced pressure and dried overnight under vacuum to obtain *tert*-butyl docosanedioyl-yGlu-OtBu (25.61g, yield 94%).

### LC-MS (Scie×100API): m/z = 611.48(M+1)⁺

### 2.3 Tert-butyl docosanedioyl-yGlu-(OSu)-OtBu

*Tert*-butyl docosanedioyl-yGlu-OtBu (25.61 g, 41.85 mmol) was dissolved in dichloromethane (250 mL) under nitrogen atmosphere, and triethylamine (17 mL) was added. The mixture was stirred for 10 min, and NHS (4.82 g, 41.85mmol) was added, followed by addition of DCC (9.50 g, 46.04 mmol). The reaction mixture was stirred at room temperature overnight. The reaction mixture was filtered, and the resulting filtrate was concentrated to almost dry. The residue was mixed with cold water and ethyl acetate, and the mixture was stirred for 20 min and subjected to liquid separation. The upper organic phase was washed with saturated brine, and after liquid separation, the upper organic phase was dried over anhydrous sodium sulfate and filtered, and the filtrate was concentrated to almost dry under reduced pressure. *tert*-butyl methyl ether was added, and the mixture was stirred for 30 min and filtered under vacuum. The filter cake was dried overnight under vacuum to obtain *tert*-butyl docosanedioyl-yGlu-(OSu)-OtBu (23.74 g, yield 80%).

### LC-MS (Scie×100API): m/z = 708.94(M+1)⁺

### 2.4 Tert-butyl docosanedioyl-γGlu-(3×OEG-OH)-OtBu

*Tert*-butyl docosanedioyl-yGlu-(OSu)-OtBu (23.74 g, 33.48 mmol) was dissolved in dichloromethane (230 mL), and the solution was stirred and added with 3× OEG (15.18 g, 33.48 mmol), triethylamine (15 mL) and water (25 mL) sequentially. The mixture was heated to obtain a clarified solution, which was then stirred at room temperature for 4 h. Then, the reaction solution was added with 10% aqueous citric acid solution (230 mL) and subjected to liquid separation. The lower organic phase was washed with saturated brine, and after liquid separation, the lower organic phase was dried over anhydrous sodium sulfate and filtered. The filtrate was concentrated to almost dryness under reduced pressure and dried overnight under vacuum to obtain *tert*-butyl docosanedioyl-γGlu-(2×OEG-OH)-OtBu (32.59 g, yield 93%).

### LC-MS (Scie×100API): m/z = 1046.70(M+1)⁺

2.5 *Tert*-butyl docosanedioyl-γGlu-(3×OEG-OSu)-OtBu *Tert*-butyl docosanedioyl-γGlu-(3×OEG-OH)-OtBu (32.59 g, 31.14 mmol) was dissolved in dichloromethane (320 mL) under nitrogen atmosphere, and triethylamine (13 mL) was added. The mixture was stirred for 10 min, and NHS (3.58 g, 31.14 mmol) was added, followed by the addition of DCC (7.07 g, 34.25 mmol). The reaction mixture was stirred at room temperature overnight. The reaction mixture was filtered, and the resulting filtrate was concentrated to almost dryness. The residue was mixed with cold water and ethyl acetate, and the mixture was stirred for 20 min and subjected to liquid separation. The upper organic phase was washed with saturated brine, and after liquid separation, the upper organic phase was dried over anhydrous sodium sulfate and filtered. The filtrate was concentrated to almost dryness under reduced pressure and dried overnight under vacuum to obtain *tert*-butyl docosanedioyl-yGlu-(3×OEG-OSu)-OtBu (32.41 g, yield 91%).

### LC-MS (Scie×100API): m/z = 1143.71(M+1)⁺

### Example 2

### A14E, B16H, B25H, B29K (N(ε)- docosanedioyl-yGlu-4xOEG), desB30 human insulin (compound 2)

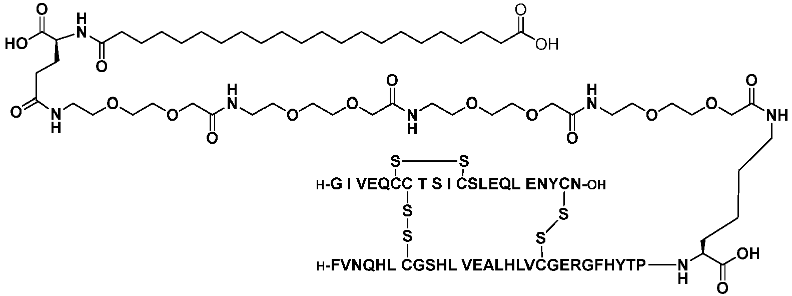

The A14E, B16H, B25H, B29K (N(ε)-docosanedioyl-γGlu-4xOEG), desB30 human insulin was prepared by procedures similar to those described in section 1 of Example 1.

### LC-MS (ESI): m/z = 1116.7136[M+6H]⁶⁺

The intermediate *tert*-butyl docosanedioyl-yGlu-(4xOEG-OSu)-OtBu was prepared by procedures similar to those described in section 2 of Example 1.

### LC-MS (Scie×100API): m/z = 1288.79(M+1)⁺

### Comparative Example 1.

### A14E, B16H, B25H, B29K(N(ε)- docosanedioyl-yGlu-12xOEG), desB30 human insulin (control compound 1)

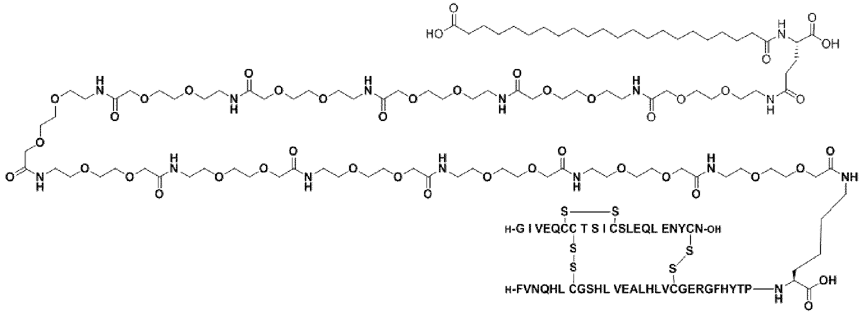

Compound A14E, B16H, B25H, B29K (N(ε)-docosyl-γGlu-12xOEG), desB30 human insulin was prepared by following procedure similar to Section 1 of Example 1.

### LC-MS(ESI): m/z=1310.1425[M+6H]⁶⁺

The intermediate *tert*-butyldocosanedioyl-yGlu-(12xOEG-OSu)-OtBu was prepared by following procedure similar to Section 2 of Example 1.

### LC-MS(Scie×100API): m/z=2451.38(M+1)⁺

### Comparative example 2

### A14E, B16H, B25H, B29K(N(ε)-eicosanedioyl-γGlu-2xOEG), desB30 human insulin (Control compound 2)

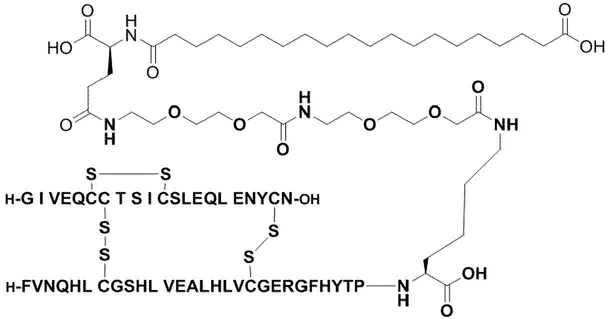

### 1, Preparation of A14E, B16H, B25H, B29K(N(ε)-eicosanedioyl-γGlu-2xOEG), desB30 human insulin

A14E, B16H, B25H, and desB30 human insulin (5g, 0.888 mmol) was dissolved in 100mM Na₂HPO₄ aqueous solution (150mL), and acetonitrile (100 mL) was added. The pH was adjusted to 10-12.5 using 1N NaOH. *Tert*-butyleicosanedioyl-γGlu-(2xOEG-OSu)-OtBu (0.948 g, 0.976 mmol) was dissolved in acetonitrile (50mL), and slowly added to the insulin solution. The pH was maintained between 10-12.5. After 120 minutes, the reaction mixture was poured into water (150mL) and the pH was adjusted to 5.0 using 1N HCl solution. Precipitate was separated by centrifugation, followed by freeze-drying. The freeze-dried crude product was added to a mixture of trifluoroacetic acid (60mL) and dichloromethane (60mL), and stirred at room temperature for 30 minutes. The mixture was concentrated to about 30 mL, poured into ice-cold n-heptane (300mL), and filtered to isolate the precipitate. The precipitate was washed twice with n- heptane. After vacuum drying, The resulting precipitate was purified by ion exchange chromatography (Ressource Q, using a gradient of 0.25%-1.25% ammonium acetate in 42.5% ethanol at pH 7.5) and reverse-phase chromatography (acetonitrile, water, TFA). The purified fractions were combined, adjusted the pH to 5.2 using 1N HCl, and separated the precipitate, which was lyophilized to obtain the control compound 2. LC-MS(electrospray): m/z=1063.6852[M+6H]⁶⁺

2, Preparation of the intermediate *tert*-butyl eicosanedioyl-yGlu-(2xOEG-OSu)-OtBu: conducted following procedure similar to step 2 of Example 1.

### 2.1 Tert-butyl eicosanedioyl-OSu

Under nitrogen protection, eicosanedioic acid *tert*-butyl ester (20 g, 50.17 mmol) and NHS (5.77 g, 50.17 mmol) was combined in dichloromethane, followed by addition of triethylamine (13.95 mL), and the resulting turbid mixture was stirred at room temperature. DCC (11.39 g, 55.19 mmol) was then added and further stirred overnight. The mixture was filtered, and the filtrate was concentrated to almost dryness. The residue was mixed with cold water and ethyl acetate, stirred for 20 minutes, and then separated. The upper organic phase was washed with saturated brine, followed by separation. The upper organic phase was dried over anhydrous sodium sulfate and filtered. The filtrate was concentrated under reduced pressure to almost dry and dried in vacuum overnight to obtain 24.12 g (97% yield) of *tert*-butyl eicosanedioyl-OSu.

### LC-MS(Scie×100API): m/z=496.36(M+1)⁺

### 2.2 Tert-butyl eicosanedioyl-yGlu-OtBu

*Tert*-butyl eicosanedioyl-OSu (24.12 g, 48.66 mmol) was dissolved in dichloromethane (250 mL) and stirred, followed by addition of H-Glu-OtBu (10.88 g, 53.53 mmol), triethylamine (12.49 mL) and water. The mixture was heated to obtain a clear solution, which was then stirred at room temperature for 4 hours. Next, 10% citric acid aqueous solution (200 mL) was added, followed by liquid-liquid extraction. The lower organic phase was washed with saturated saline solution, and then dried over anhydrous sodium sulfate. After filtration, the filtrate was concentrated under reduced pressure to almost dry, and dried overnight under vacuum. 27.27 g (96% yield) of *tert*-butyl eicosanedioyl-yGlu-OtBu was obtained. LC-MS (Sciex 100 API): m/z = 584.44 (M+1)⁺

### 2.3 Tert-butyl eicosanedioyl-yGlu-(OSu)-OtBu

Under nitrogen protection, *tert*-butyl eicosanedioyl-yGlu-OtBu (27.27 g, 46.71 mmol) was dissolved in dichloromethane (300 mL). Triethylamine (11.99 mL) was added and stirred for 10 minutes. NHS (5.38 g, 50.17 mmol) was then added, followed by addition of DCC (10.60 g, 51.38 mmol). The mixture was stirred overnight at room temperature. After filtration, the filtrate was concentrated to almost dry, and the residue was mixed with cold water and ethyl acetate, stirred for 20 minutes, and then separated. The upper organic phase was washed with saturated saline solution, dried over anhydrous sodium sulfate, and filtered. The filtrate was concentrated under reduced pressure to almost dry, and methyl *tert*-butyl ether was added, followed by stirring for 30 minutes. The resulting mixture was filtered and dried under vacuum overnight. 25.76 g (81% yield) of *tert*-butyl eicosanedioyl-yGlu-(OSu)-OtBu was obtained.

### LC-MS(Scie×100API): m/z=681.46(M+1)⁺

### 2.4 Tert-butyl eicosanedioyl-yGlu-(2xOEG-OH)-OtBu

*Tert*-butyl eicosanedioyl-yGlu-(OSu)-OtBu (25.76 g, 37.83 mmol) was dissolved in dichloromethane (250 mL) and stirred, followed by addition of 2xAEEA (11.66 g, 37.83 mmol), triethylamine (9.71 mL) and water (25 mL). The mixture was heated to obtain a clear solution, which was then stirred at room temperature for 4 hours. Next, 10% citric acid aqueous solution (200 mL) was added, followed by liquid-liquid extraction. The lower organic phase was washed with saturated saline solution, dried over anhydrous sodium sulfate, and filtered. The filtrate was concentrated under reduced pressure to almost dry and dried under vacuum overnight. 30.75 g (93% yield) of *tert*-butyl eicosanedioyl-yGlu-(2xOEG-OH)-OtBu was obtained.

### LC-MS (Sciex 100 API): m/z = 874.59 (M+1)⁺

### 2.5 Tert-butyl eicosanedioyl-yGlu-(2xOEG-OSu)-OtBu

Under nitrogen protection, *tert*-butyl eicosanedioyl-yGlu-(2xOEG-OH)-OtBu (30.75 g, 35.18 mmol) was dissolved in dichloromethane (300 mL), followed by addition of triethylamine (9.03 mL) and stirring for 10 min. NHS (4.05 g, 35.18 mmol) was added, followed by DCC (7.98 g, 38.70 mmol). The mixture was stirred overnight at room temperature. After filtration, the filtrate was concentrated to almost dry, and the residue was mixed with cold water and ethyl acetate, stirred for 20 minutes, and then separated. The upper organic phase was washed with saturated saline solution, dried over anhydrous sodium sulfate, and filtered. The filtrate was concentrated under reduced pressure to almost dry, and dried under vacuum overnight. 31.09 g (91% yield) of *tert*-butyl eicosanedioyl-yGlu-(2xOEG-OSu)-OtBu was obtained.
LC-MS(Scie×100API): m/z=971.61(M+1)⁺
LC-MS(electrospray): m/z=1400.68[M+5H]⁵⁺

### Experimental Example 1: Pharmacokinetics

The purpose of this example is to illustrate the *in vivo* pharmacokinetic properties of the compounds in the present invention.

### Pharmacokinetics in Beagle Dogs

Twelve female Beagle dogs were divided into four groups (each group containing 3 dogs): Control Compound 1 subcutaneous (SC) group, Compound 1 SC group, Control Compound 1 intravenous (IV) group, and Compound 1 IV group. The dogs were administered 17 nmol/kg of control compound 1 and compound 1 via subcutaneous injection, and 1.6 nmol/kg of control compound 1 and Compound 1 via intravenous injection. Blood samples were collected from each group before administration (0 minute), at 1 hour, 3 hours, 6 hours, 9 hours, 12 hours, 24 hours, 32 hours, 48 hours, 72 hours, 96 hours, 120 hours, 144 hours, and 168 hours after administration to measure blood drug concentrations. Pharmacokinetic parameters including T_{1/2}, Tₘₐₓ, Cₘₐₓ, AUCₗₐₛₜ, AUC_{Inf}, and MRT were calculated using WinNonLin V8.2 software. The experimental results are summarized in Table 1.

**Table 1: Average pharmacokinetic Parameters of Control Compound 1 and Compound 1 injected in Beagle Dogs**

| | Compound 1-subcutaneous (Mean±SEM) | Compound 1-intravenous (Mean±SEM) | Control Compound 1-subcutaneous Mean±SEM) | Control Compound 1-intravenous (Mean±SEM) |
|---|---|---|---|---|
| T_{1/2}(h) | 38.8±2.5 | 38.9±6.3 | 22.6±0.5 | 16.5±2.2 |
| Tₘₐₓ(h) | 26.7±2.7 | - | 22.7±5.8 | - |
| Cₘₐₓ(ng/mL) | 953±104.5 | 251±2.9 | 566±40.5 | 284±1.3 |
| AUCₗₐₛₜ(h*ng/mL) | 71882±9774.1 | 5461±200.4 | 34407±1631.2 | 2896±120.3 |
| AUC_{Inf}(h*ng/mL) | 76907±10570.8 | 5642±210.6 | 34691±1592.8 | 3256±207.7 |
| MRT(h) | 68.1±1.9 | 41.0±2.6 | 45.8±2.6 | 20.0±1.5 |
| F(%) | 125±16.9 | - | 102±5.6 | - |

| | | | | |
|---|---|---|---|---|
| T_{1/2}= Terminal elimination half-life, Tₘₐₓ= Time corresponding to the maximum measured blood drug concentration, Cₘₐₓ= Maximum measured blood drug concentration, AUC₀₋ₗₐₛₜ= Area under the blood drug concentration-time curve from time zero to the last time point, AUC_{Inf}= Area under the blood drug concentration-time curve from time zero to infinity, MRT= Mean residence time, F= Bioavailability | | | | |

From the above experimental results, it can be observed that in Beagle dogs, compared to control compound 1, compound 1 of the present invention exhibits a longer half-life, which is nearly double that of control compound 1 when administered subcutaneously and more than double that of control compound 1 when administered intravenously. The compound 1 has higher bioavailability; higher exposure level *in vivo,* which is more than double that of control compound 1; and longer mean residence time.

### Experimental Example 2.

Pharmacological Study in Streptozotocin (STZ)-Induced Type 1 Diabetes Mellitus (T1DM) Rats

The purpose of this study is to confirm the regulatory effect of acylated insulin of the present invention on blood glucose (BG) in STZ-induced T1DM rats.

Forty STZ-induced T1DM rats (male and female in equal numbers) were divided into four groups with 10 rats in each group based on the weight/random blood glucose: Vehicle group, Compound 1 low dose group, Compound 1 high dose group, and Control Compound 2 high dose group. Each group of T1DM rats underwent the following treatments: subcutaneous injection of vehicle (Vehicle group); or subcutaneous injection of high dose Control Compound 2 (Control Compound 2 high dose group) and Compound 1 (Compound 1 high dose group) at dosages of 12U/kg for the first to second doses and 18U/kg for subsequent 3-10 doses; or subcutaneous injection of low dose Compound 1 (Compound 1 low dose group) at dosages of 6U/kg for the first 2 doses and 9U/kg for subsequent 3-10 doses. The vehicle comprised 45mM phenol, 10mM *m*-cresol, 15mg/ml glycerol, 5mM disodium phosphate, 20mM sodium chloride, with a pH of 7.4.

For the Control Compound 2 high dose group and Compound 1 high dose group, the compound 1 and control compound 2 were respectively dissolved in vehicle to achieve a dosage of 12U/ml during the first to second administration and 18U/ml during the 3^{rd} to 10^{th} administration, and the dosage volume was 1ml/kg. For the low dose group, compound 1 was dissolved in vehicle to achieve a dosage of 6U/ml during the first to second administration and 9U/ml during the 3^{rd} to 10^{th} administration, and the dosage volume was 1ml/kg. Subcutaneous administration was performed on days 1, 4, 7, 11, 14, 17, 20, 23, 26, and 29, with blood glucose measured before the first dose, and at 3 hours, 6 hours, 9 hours, 24 hours, 48 hours, and 72 hours post-first dose. Random blood glucose was then measured daily before the last dose, and at 3 hours, 6 hours, 9 hours, 12 hours, 24 hours, 32 hours, 48 hours, 72 hours, 96 hours, and 120 hours post-last dose. For each single dose of acylated insulin, dose-response curves for blood glucose over time were plotted for days 1-27 and post-last dose, and the corresponding area under the blood glucose-time curve (ΔAUC) was calculated. On the 18th day, whole blood was collected in EDTA anticoagulant to measure the percentage of glycated hemoglobin (Hb1Ac).

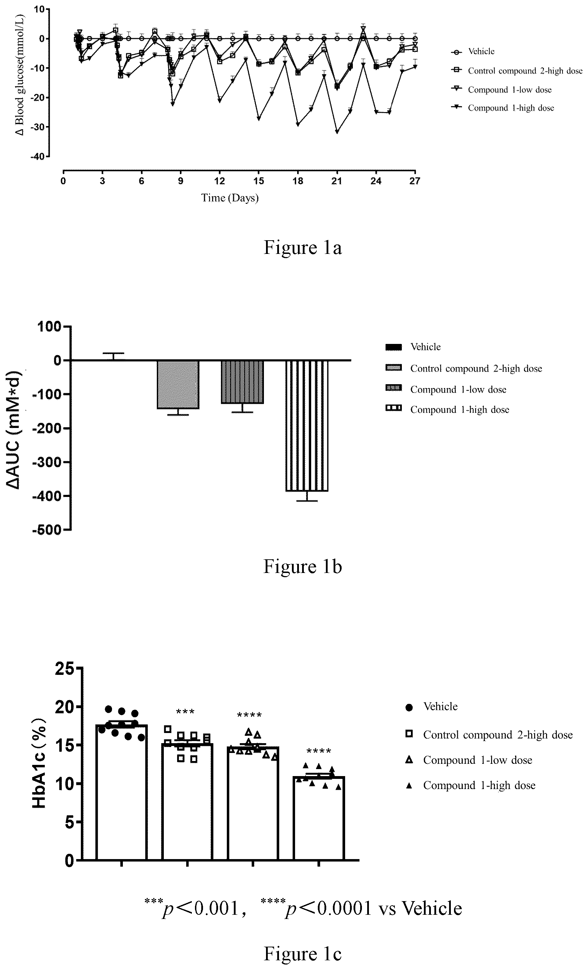

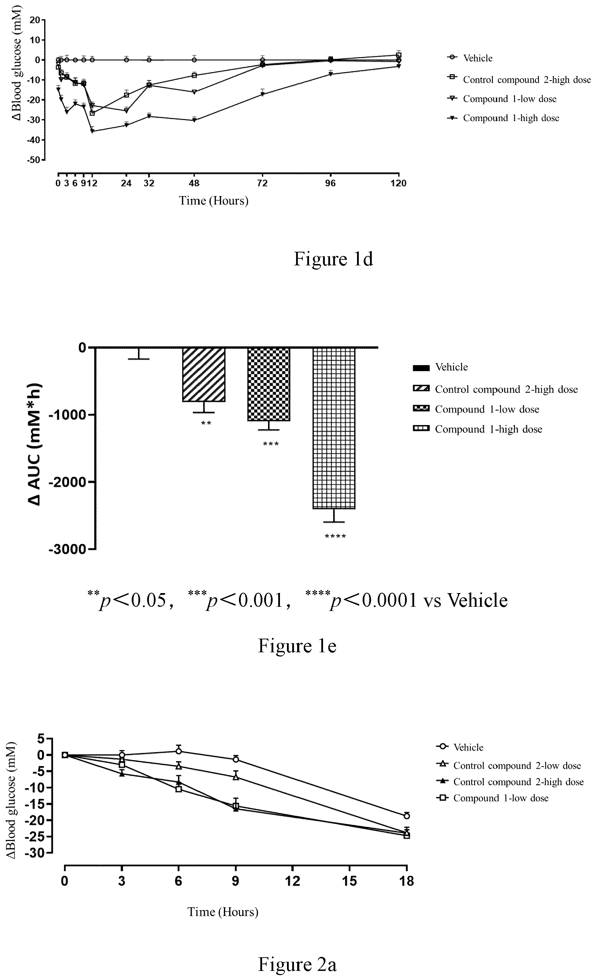

The experimental results depicted in Figures 1a-1e demonstrate that the hypoglycemic effect and reduction in Hb1Ac achieved by Compound 1 of the present invention in T1DM rats are significantly superior to those of Control Compound 2. Even the dosage of compound 1 is at half the dosage of Control Compound 2, Compound 1 exhibits comparable or superior hypoglycemic and Hb1Ac reduction effects.

### Experimental Example 3

### Pharmacological Study in db/db Mice

The purpose of this study is to confirm the regulatory effect of compound 1 and control compound 2 on blood glucose (BG) under diabetic conditions.

Male db/db (BKS/Lepr) mice aged 8-9 weeks were housed in barrier facilities in appropriate-sized cages with free access to standard food and purified water. Environmental conditions were controlled at a relative humidity of 40%-60% and a temperature of 22°C-24°C. After a 1-2 weeks adaptation period, the mice were used for experiments.

24 male db/db mice were divided into four groups with 6 mice in each group based on weight/random blood glucose: vehicle group, Compound 1 low dose group, Control Compound 2 low dose group, Control Compound 2 high dose group. The db/db mice in each group were treated as follows: subcutaneous injection of vehicle (Vehicle group); or subcutaneous injection of low dose compound 1 (Compound 1 low dose group) and low dose control compound 2 (Control Compound 2 low dose group) at a dosage of 54 nmol/kg for both; or subcutaneous injection of high dose control compound 2 (Control Compound 2 high dose group) at a dosage of 108 nmol/kg. The vehicle contained 45mM phenol, 10mM *m*-cresol, 15mg/ml glycerol, 5mM disodium hydrogen phosphate, 20mM sodium chloride, with a pH of 7.4. In the Compound 1 and Control Compound 2 low dose groups, compound 1 and control compound 2 were dissolved in vehicle to achieve a dosage concentration of 10.8nmol/ml, administered at a volume of 5 ml/kg; in the Control Compound 2 high dose group, control compound 2 was dissolved in vehicle to achieve a dosage concentration of 21.6nmol/ml, administered at a volume of 5ml/kg. Subcutaneous administration was performed once, with animals fasted during dosing. The fasting glucose levels were assessed at 3, 6, 9, and 18 hours post-dosing. To simulate feeding, an oral glucose tolerance test (OGTT) was conducted after the 18-hour blood glucose measurement in the experiment. Mice were orally administered with a 5% glucose solution (50mg/mL, 10mL/kg), and blood glucose was measured at 15min, 30min, 60min, and 120min post administration. After the 21.5-hour blood glucose measurement, a second OGTT was performed, and the study was terminated after the 23.5 hours blood glucose assessment.

The mice tail was cleaned with an alcohol cotton ball, and blood drops were collected from the tail using a disposable blood collection needle. Blood glucose was measured using a glucose meter and its matching test strips (Roche). Dose-response curves for blood glucose over time were plotted for each single dose of acylated insulin, and the area under the blood glucose-time curve (AUC) or the area difference under the blood glucose-time curve (ΔAUC) from time zero to the end of monitoring was calculated.

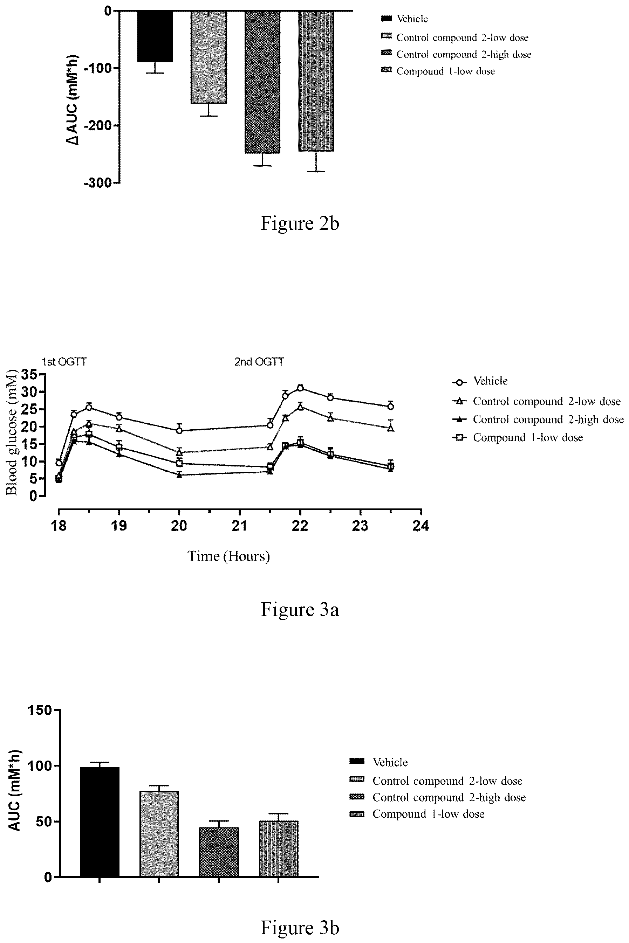

The experimental results depicted in Figures 2a-3b demonstrate that compound 1 of the present invention has a significantly superior hypoglycemic effect than control compound 2 in db/db mice. When the dose of compound 1 was half that of control compound 2, compound 1 still exhibits a comparable hypoglycemic effect to that of Control Compound 2.

### Experimental Example 4

### Pharmacological Study in streptozotocin (STZ)-Induced Type 1 Diabetic Mellitus (T1DM) Rats

Following similar experimental procedures as in Experimental Example 2, a pharmacological study was conducted in streptozotocin (STZ)-Induced Type 1 Diabetic Mellitus (T1DM) Rats
24 male rats with STZ-induced T1DM were divided into three groups with 8 rats in each group based on weight/random blood glucose: vehicle group, Control Compound 2 group, Compound 1 group. The rats in each group were treated as follows: subcutaneous injection of vehicle (Vehicle group); subcutaneous injection of control compound 2 and compound 1 at a dosage of 18 U/kg for the first to third doses and 10 U/kg for 4th to 6th doses; the vehicle consists of 45mM phenol, 10mM *m*-cresol, 15mg/ml glycerol, 5mM disodium hydrogen phosphate, 20mM sodium chloride, with a pH of 7.4.

For the first to third administration, compound 1 and control compound 2 were dissolved in vehicle to achieve a dosage of 18U/ml (108 nmol/ml), administered at a volume of 1ml/kg; for the 4th to 6th administration, compound 1 and control compound 2 were dissolved in vehicle to achieve a dosage concentration of 10U/ml (60nmol/ml), administered at a volume of 1ml/kg. Subcutaneous administration (s.c.) was performed on days 0, 3, 6, 9, 12, 15 (with dose adjustment to 10 U/ml (60 nmol/ml) starting from day 9), and blood glucose was measured before the first administration, and at 3, 6, 9, 24, 48, and 72 hours post-first administration, followed by daily measurements until day 18. Dose-response curves for blood glucose over time were plotted for each single dose of acylated insulin, and the area difference under the blood glucose-time curve (ΔAUC) from time zero to the end of monitoring was calculated. On the 15th day, the percentage of glycosylated hemoglobin (Hb1Ac) in whole blood was detected by EDTA anticoagulation.

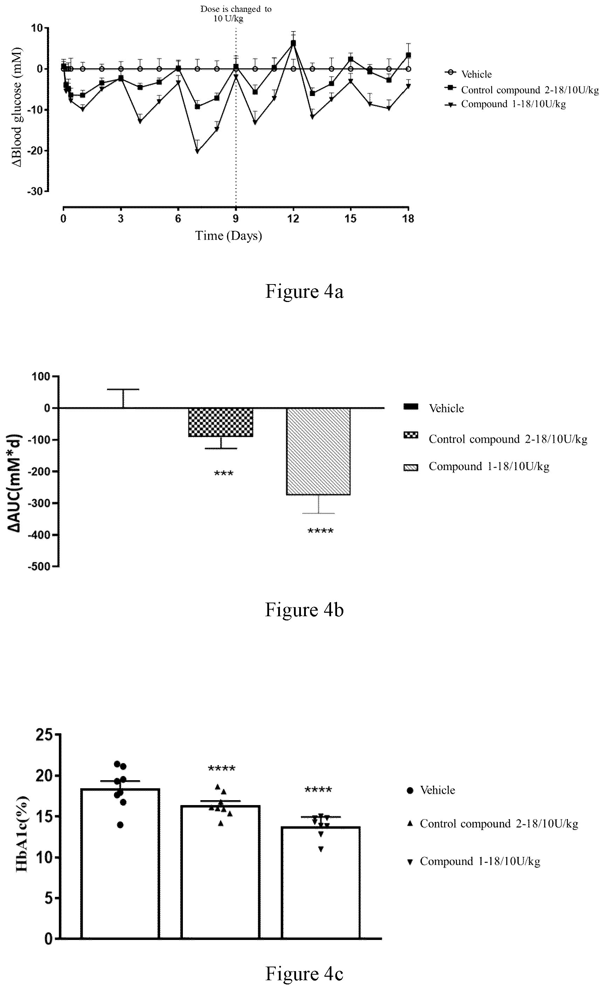

The experimental results depicted in Figures 4a-4c demonstrate that compound 1 of the present invention has significantly superior hypoglycemic and Hb1Ac-lowering effects than control compound 2 in T1DM rats.

### Experimental Example 5

### Pharmacokinetic Study of Compound 1 Formulations with Different Zinc Ion Content in Beagle Dogs

10 male Beagle dogs were divided into two groups with 5 dogs in each group: Formulation 1 group of compound 1(The Zn content is 2.3 moles of zinc ions/6 moles of acylated insulin) and Formulation 2 group of compound 1 (The Zn content is 4.8moles of zinc ions/6 moles of acylated insulin), administered by subcutaneous injection of Formulation 1 and Formulation 2 at a dosage of 17nmol/kg of Compound 1 respectively. The other components of Formulation 1 and Formulation 2 were 5mM disodium hydrogen phosphate, 45mM phenol, 10mM *m*-cresol, 20mM sodium chloride, and 17mg/ml glycerol. Blood samples were collected from the Formulation 1 group and Formulation 2 group at pre-dose (0min) and post-dose at 1, 3, 6, 9, 12, 24, 32, 48, 72, 96, 120, 144, and 168 hours to measure blood drug concentrations. Pharmacokinetic parameters including T_{1/2}, Tₘₐₓ, Cₘₐₓ, AUCₗₐₛₜ, AUC_{Inf}, AUC_{-%Extrap-obs} (%), and MRT were calculated using WinNonLin V8.2 software, and the experimental results are summarized in Table 2.

**Table 2: Mean Pharmacokinetic Parameters of Compound 1-1 and Compound 1-2 Administered Subcutaneously in Beagle Dogs**

| | Formulation 1 (Mean±SD) | Formulation 2 (Mean±SD) |
|---|---|---|
| T_{1/2}(h) | 31.5±5.6 | 35.6±5.1 |
| Tₘₐₓ(h) | 13.8±5.8 | 13.8±9.4 |
| Cₘₐₓ(ng/mL) | 650±129 | 856±218 |
| AUCₗₐₛₜ(h*ng/mL) | 47161±6956 | 54096±8152 |
| AUC_{Inf}(h*ng/mL) | 48981±7526 | 56569±9159 |
| AUC_{_%Extrap_obs} (%) | 3.63±0.8 | 4.25±1.22 |
| MRT(h) | 58.5±3.5 | 56.7±3.7 |

| | | |
|---|---|---|
| T_{1/2}= Terminal elimination half-life, Tₘₐₓ= Time corresponding to the maximum observed blood drug concentration, Cₘₐₓ= Maximum observed blood drug concentration, AUCₗₐₛₜ= Area under the blood drug concentration-time curve from time zero to the last measurable time point, AUC_{Inf}= Area under the blood drug concentration-time curve from time zero to infinity, AUC_{_%Extrap_obs}=the proportion of AUC from the last observed point to theoretical extrapolation to infinity relative to AUC_{INF}, MRT= Mean residence time | | |

Based on the above experimental results, it can be observed that the Compound 1 formulation exhibits a longer half-life, higher exposure level, and extended residence time in beagle dogs at the above zinc ion concentration range.

### Experimental Example 6

The purpose of this experiment is to measure the chemical stability of the acylated insulin formulations of the present invention.

### Acylated Insulin Formulations

Disodium Hydrogen Phosphate Buffer Formulation: Dissolve the title compound 1 of Example 1 in a disodium hydrogen phosphate solution with a final concentration of 5mM. Mix phenol, *m*-cresol, glycerol, and sodium chloride according to the quantities in Tables 3, 5, 6, then add the mixture to the compound 1 solution and adjust the pH to the values specified in the table. Finally, add zinc acetate slowly and adjust the pH to the final value. This produces acylated insulin formulations with final insulin concentrations as shown in the table below, where the Zn content is expressed as Zn/6 moles of acylated insulin (abbreviated as "Zn/6ins"). Citric Acid Buffer Formulation: Dissolve the title compound 1 of Example 1 in a citric acid monohydrate solution with a final concentration of 0.5mg/ml. Mix phenol, *m*-cresol, glycerol, and sodium chloride according to the quantities in Table 4, then add the mixture to the compound 1 solution and slowly add zinc acetate to adjust the pH to the final value. This produces acylated insulin formulations with final insulin concentrations as shown in the table below, where the Zn content is expressed as Zn/6 moles of acylated insulin (abbreviated as "Zn/6ins"). The chemical stability of the formulations in this example can be assessed by measuring changes of high molecular weight proteins (HMWP) and active substances relative to day 0 after storage at 25°C and 37°C for 14 days and 35 days.

### Measurement of High Molecular Weight Proteins (HMWP)

High molecular weight proteins (HMWP) content is determined by high-performance liquid chromatography (HPLC) using a ShodexTM PROTEIN KW-802.5 column (8.0mM × 300mM) at a column temperature of 30°C and sample cell temperature of 5°C, with mobile phase at a flow rate of 0.5ml/min. The mobile phase consists of 3L of 0.1% arginine solution, 750ml of ice acetic acid, and 1250ml of acetonitrile. Detection wavelength is set at 276 nm with an injection volume of 3µl. Tables 3-6 present the increase in HMWP relative to day 0 after storage at 25°C and 37°C for 14 days and 35 days.

**Table 3: Detection Results of the Increased Amount of HMWP**

| 2.1mM compound 1 10mM *m*-cresol 17mg/ml glycerol 55mM phenol 20mM NaCl 5mM Na₂HPO₄ pH 7.4 | 25°C The increase in the amount of HMWP on day 14 relative to day 0 (%) | 25°C The increase in the amount of HMWP on day 35 relative to day 0 (%) | 37°C The increase in the amount of HMWP on day 14 relative to day 0 (%) | 37°C The increase in the amount of HMWP on day 35 relative to day 0 (%) |
|---|---|---|---|---|
| 2.2 Zn/6ins | 0.12 | 0.38 | 0.48 | 1.56 |
| 3 Zn/6ins | 0.33 | 0.42 | 0.71 | 1.70 |
| 3.7 Zn/6ins | 0.09 | 0.28 | 0.36 | 1.22 |
| 4.8 Zn/6ins | 0.08 | 0.17 | 0.33 | 0.97 |
| 5.6 Zn/6ins | 0.05 | 0.13 | 0.24 | 0.52 |

**Table 4: Detection Results of the Increased Amount of HMWP**

| 2.1mM compound 1 2.3 Zn/6ins 17mg/ml glycerol 45mM phenol 20mMNaCl 0.5mg/ml citric acid pH 7.4 | 25°C The increase in the amount of HMWP on day 14 relative to day 0 (%) | 25°C The increase in the amount of HMWP on day 35 relative to day 0 (%) | 37°C The increase in the amount of HMWP on day 14 relative to day 0 (%) | 37°C The increase in the amount of HMWP on day 35 relative to day 0 (%) |
|---|---|---|---|---|
| 0mM *m*-cresol | 0.05 | 0.17 | 0.2 | 0.53 |
| 5mM *m*-cresol | 0.07 | 0.16 | 0.2 | 0.56 |
| 10mM *m*-cresol | 0.06 | 0.19 | 0.22 | 0.61 |

**Table 5: Detection Results of the Increased Amount of HMWP**

| 2.1mM compound 1 2.3 Zn/6ins 17mg/ml glycerol 45mM phenol 20mM NaCl 10mM *m*-cresol 5mM Na₂HPO₄ | 25°C The increase in the amount of HMWP on day 14 relative to day 0 (%) | 25°C The increase in the amount of HMWP on day 35 relative to day 0 (%) | 37°C The increase in the amount of HMWP on day 14 relative to day 0 (%) | 37°C The increase in the amount of HMWP on day 35 relative to day 0 (%) |
|---|---|---|---|---|
| pH 6.5 | 0.15 | 0.3 | 0.69 | 1.7 |
| pH 6.8 | 0.09 | 0.4 | 0.68 | 1.6 |
| pH 7.0 | 0.21 | 0.4 | 0.73 | 1.7 |
| pH 7.2 | 0.22 | 0.4 | 0.74 | 1.7 |
| pH 7.4 | 0.27 | 0.4 | 0.68 | 1.7 |
| pH 7.6 | 0.19 | 0.4 | 0.8 | 1.9 |
| pH 7.8 | 0.18 | 0.3 | 0.81 | 2.4 |
| pH 8.0 | 0.14 | 0.3 | 0.95 | 3.0 |

**Table 6: Detection Results of the Increased Amount of HMWP**

| 2.1mM compound 1 2.2 Zn/6ins 17mg/ml glycerol 10mM *m*-cresol 20mM NaCl 5mM Na₂HPO₄ pH 7.4 | 25°C The increase in the amount of HMWP on day 14 relative to day 0 (%) | 25°C The increase in the amount of HMWP on day 35 relative to day 0 (%) | 37°C The increase in the amount of HMWP on day 14 relative to day 0 (%) | 37°C The increase in the amount of HMWP on day 35 relative to day 0 (%) |
|---|---|---|---|---|
| 35mM phenol | 0.16 | 0.32 | 0.55 | 1.4 |
| 45mM phenol | 0.22 | 0.43 | 0.71 | 1.8 |
| 55mM phenol | 0.21 | 0.42 | 0.75 | 1.8 |

Based on the above table, it can be observed that the amount of HMWP in the acylated insulin formulation of the present invention increases very slowly over time within the ranges of zinc ion concentration, *m-*cresol concentration, pH value, and phenol concentration above. This indicates that the acylated insulin formulation of the present invention exhibits excellent chemical stability within the ranges of zinc ion concentration, *m*-cresol concentration, pH value, and phenol concentration above.

### Measurement of the amount of active substance

The content of insulin active substance was determined by high-performance liquid chromatography (HPLC) using a Waters Kromasil 100A-3.5µm-C4 column (4.6* 150mm) at a column temperature of 35°C and a sample chamber temperature of 5°C, with eluent at a flow rate of 1.0ml/min. The eluent was composed of the following mobile phases:
Phase A consisted of 4.5L of 0.2M anhydrous sodium sulfate solution and 0.5L of acetonitrile, with pH adjusted to 2.3 using concentrated phosphoric acid.
Phase B was 75% acetonitrile (v/v).

The gradient profile was as follows:
0-40min: isocratic gradient of 64% / 36% A / B, 40-65min: linear change of 0% / 100% A / B, 50-51min: linear change of 64% / 36% A / B, 65-70min: isocratic gradient of 64% / 36% A / B. Tables 7-10 present the reduction in the amount of active substance relative to day 0 on day 14 and day 35 day at 25°C and 37°C.

**Table 7 Detection Results of the Decrease in the Amount of Active Substance**

| 2.1mM compound 1 10mM *m*-cresol 17mg/ml glycerol 55mM phenol 20mM NaCl 5mM Na₂HPO₄ pH 7.4 | 25°C The decrease in the amount of HMWP on day 14 relative to day 0 (%) | 25°C The decrease in the amount of HMWP on day 35 relative to day 0 (%) | 37°C The decrease in the amount of HMWP on day 14 relative to day 0 (%) | 37°C The decrease in the amount of HMWP on day 35 relative to day 0 (%) |
|---|---|---|---|---|
| 2.2 Zn/6ins | 0.22 | 1.57 | 1.35 | 4.43 |
| 3 Zn/6ins | 0.16 | 0.47 | 1.34 | 3.06 |
| 3.7 Zn/6ins | 0.22 | 1.55 | 1.09 | 4.02 |
| 4.8 Zn/6ins | 0.18 | 1.41 | 1.09 | 3.97 |
| 5.6 Zn/6ins | 0.16 | 1.31 | 0.9 | 3.67 |

**Table 8: Detection Results of the Decrease in the Amount of Active Substance**

| 2.1mM compound 1 2.3 Zn/6ins 17mg/ml glycerol 45mM phenol 20mM NaCl 0.5mg/ml citric acid pH 7.4 | 25°C The decrease in the amount of HMWP on day 14 relative to day 0 (%) | 25°C The decrease in the amount of HMWP on day 35 relative to day 0 (%) | 37°C The decrease in the amount of HMWP on day 14 relative to day 0 (%) | 37°C The decrease in the amount of HMWP on day 35 relative to day 0 (%) |
|---|---|---|---|---|
| 0mM *m*-cresol | 0.23 | 0.47 | 0.87 | 2.08 |
| 5mM *m*-cresol | 0.21 | 0.53 | 0.83 | 2.06 |
| 10mM *m*-cresol | 0.18 | 0.56 | 0.88 | 2.11 |

**Table 9: Detection Results of the Decrease in the Amount of Active Substance**

| 2.1mM compound 1 2.3 Zn/6ins 17mg/ml glycerol 45mM phenol 20mM NaCl 10mM *m*-cresol 5mM Na₂HPO₄ | 25°C The decrease in the amount of HMWP on day 14 relative to day 0 (%) | 25°C The decrease in the amount of HMWP on day 35 relative to day 0 (%) | 37°C The decrease in the amount of HMWP on day 14 relative to day 0 (%) | 37°C The decrease in the amount of HMWP on day 35 relative to day 0 (%) |
|---|---|---|---|---|
| pH 6.5 | 0.34 | 0.62 | 1.51 | 3.6 |
| pH 6.8 | 0.34 | 0.6 | 1.41 | 3.31 |
| pH 7.0 | 0.33 | 0.57 | 1.46 | 3.28 |
| pH 7.2 | 0.38 | 0.62 | 1.46 | 3.29 |
| pH 7.4 | 0.37 | 0.6 | 1.43 | 3.38 |
| pH 7.6 | 0.41 | 0.61 | 1.64 | 3.71 |
| pH 7.8 | 0.32 | 0.52 | 1.72 | 4.15 |
| pH 8.0 | 0.71 | 0.65 | 1.96 | 5.14 |

**Table 10: Detection Results of the Decrease in the Amount of Active Substance**

| 2.1mM compound 1 2.2 Zn/6ins 17mg/ml glycerol 10mM *m*-cresol 20mM NaCl 5mM Na₂HPO₄ pH 7.4 | 25°C The decrease in the amount of HMWP on day 14 relative to day 0 (%) | 25°C The decrease in the amount of HMWP on day 35 relative to day 0 (%) | 37°C The decrease in the amount of HMWP on day 14 relative to day 0 (%) | 37°C The decrease in the amount of HMWP on day 35 relative to day 0 (%) |
|---|---|---|---|---|
| 35mM phenol | 0.17 | 0.46 | 1.4 | 3.12 |
| 45mM phenol | 0.62 | 0.86 | 1.44 | 3.75 |
| 55mM phenol | 0.27 | 0.56 | 1.56 | 3.41 |

From the above table, it can be observed that the amount of active substance in the acylated insulin formulation of the present invention changes very slowly over time within the ranges of zinc ion concentration, cresol concentration, pH value, and phenol concentration above. Therefore, all the acylated insulin formulations of the present invention exhibit excellent chemical stability.

### Comparative example 3

### B29K(N(ε)-octadecanedioyl-γGlu-2xOEG), desB30 human insulin (control compound 3)

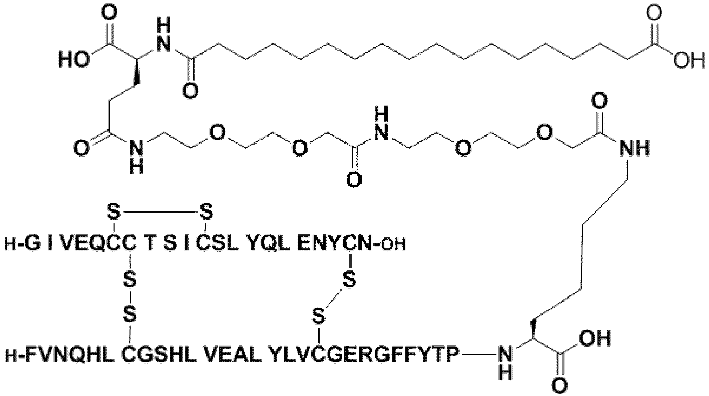

### 1. Synthesis of des(B30) human insulin

Des(B30) human insulin was prepared according to the method described in Example 11 of Chinese patent CN1056618C.

### 2. Preparation of target insulin

DesB30 human insulin (5g, 0.876mmol) was dissolved in 100mM Na₂HPO₄ aqueous solution (150mL) and acetonitrile (100mL) was added. The pH was adjusted to 10-12.5 with 1 N NaOH. *Tert*-butyl octadecanedioyl-γGlu-(2xOEG-OSu)-OtBu(0.91g , 0.964mmol) was dissolved in acetonitrile (50mL), and the solution was slowly added to the insulin solution. The pH was maintained at 10-12.5. After 120min, the reaction mixture was added to water (150mL), and the pH was adjusted to 5.0 with 1 N aqueous HCl solution. The precipitate was separated out by centrifugation and lyophilized. The crude product was added to a mixed solution of trifluoroacetic acid (60mL) and dichloromethane (60mL), and the mixture was stirred at room temperature for 30min. The mixture was then concentrated to about 30mL and poured into ice-cold n-heptane (300mL), and the precipitated product was isolated by filtration and washed twice with n-heptane. The resulting precipitate was dried under vacuum and purified by ion exchange chromatography (Resource Q, 0.25%-1.25% ammonium acetate gradient in 42.5% ethanol, pH 7.5) and reverse phase chromatography (acetonitrile, water, TFA). The purified fractions were combined, adjusted to pH 5.2 with 1 N HCl, and separated to obtain the precipitate, which was lyophilized to obtain the control compound 3.

### LC-MS(electrospray): m/z=1284.61[M+5H]⁵⁺

### 3, Preparation of intermediate tert-butyl octadecanedioyl-γGlu-(2×OEG-OSu)-OtBu

Intermediate *tert*-butyl octadecanedioyl-γGlu-(2xOEG-OSu)-OtBu was synthesized following a procedure similar to Step 2 of Comparative Example 1.

### LC-MS(Scie×100API): m/z=942.58(M+1)⁺

### Comparative example 4

### B29K(N(ε)-octadecanedioyl-γGlu-6xOEG), desB30 human insulin (control compound 4)

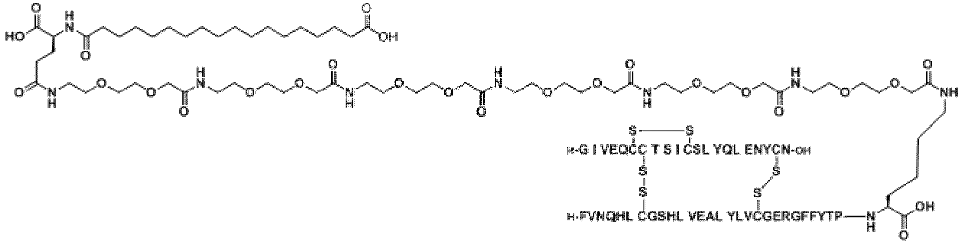

Control compound 4 was synthesized following a procedure similar to Step 2 of Comparative Example 3.

### LC-MS(electrospray): m/z=1400.68[M+5H]⁵⁺

### Comparative Example 5: Pharmacological Study in db/db Mice

The purpose of this study was to confirm the regulatory effect of the acylated insulin of the present invention on blood glucose (BG) under diabetic conditions.

The control compounds from Comparative Examples 3 and 4 were tested in a single-dose study in obese diabetic mouse models (db/db mice). The acylated insulin was tested at a dose of 9 U/kg to assess its glucose-lowering effect.

Male db/db (BKS/Lepr) mice aged 8-9 weeks were housed in barrier facilities in appropriate-sized cages with access to standard food and purified water. Environmental conditions were controlled at a relative humidity of 40%-60% and a temperature of 22°C-24°C. After an adaptation period of 1-2 weeks, the mice were used for experiments. Baseline blood glucose was assessed and mice were weighed before the experiment. Mice were matched and randomly assigned to either the solvent group or the treatment group, receiving the following treatments: subcutaneous injection of solvent or subcutaneous injection of acylated insulin at 9 U/kg. The solvent contained: glycerol 19.6mg/ml, phenol 1.5mg/ml, *m*-cresol 1.72mg/ml, zinc ion concentration 55 µg/ml, and pH 7.6.

The acylated insulin was dissolved in the solvent to a concentration of 1.8 U/ml at a volume of 5ml/kg (that is, 50µl/10g body weight). Subcutaneous administration (S.C.) was performed with a single injection at the nape of the neck around 10:30 am (time 0). During dosing, animals were fasted but allowed free access to water. Blood glucose levels were assessed in mice at 3, 6, 9, 12, and 15 hours post-dosing. To simulate feeding, an oral glucose tolerance test (OGTT) was started after the 15-hour blood glucose measurement, administering glucose solution (100mg/mL, 10mL/kg) by gavage, and measuring blood glucose at 30 min, 60 min, 120 min, and 180 min. The OGTT was conducted three times consecutively. Based on preliminary experiment results, the efficacy of the test compound was nearly absent, and terminated after evaluating blood glucose at 30 hours when the final OGTT was conducted.

The mouse tail was cleaned with alcohol cotton ball. Blood was collected using a disposable blood collection needle from the tail. Blood glucose was measured using a glucometer and compatible test strips (Roche). A dose-response curve of blood glucose over time was plotted for each individual dose of acylated insulin.

To demonstrate the effect of the acylated insulin of the present invention on blood glucose, the area under the blood glucose-time curve (AUC) from 0 to the monitoring endpoint was calculated for each individual dose-response curve. A smaller AUC value indicates a better glucose-lowering effect and greater efficacy.

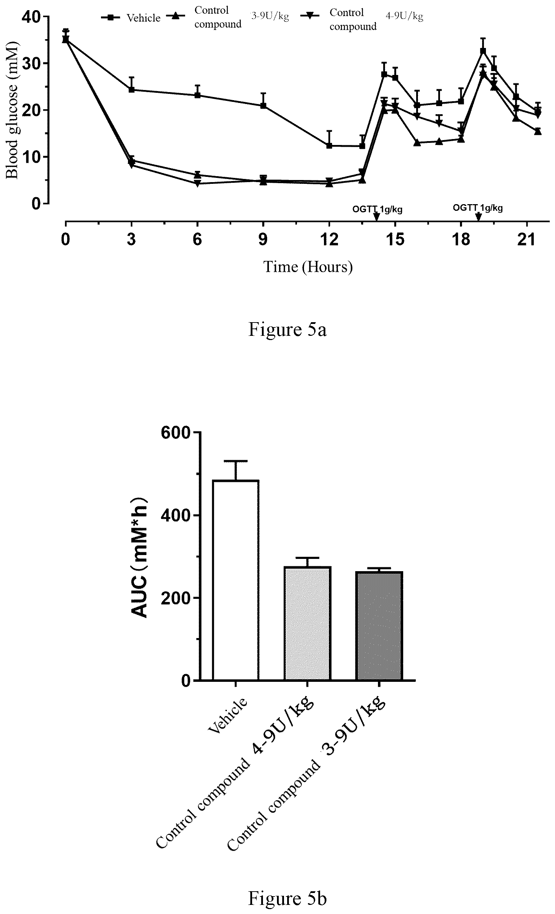

Figures 5a and 5b show that the glucose-lowering effects of control compounds 3 and 4 are comparable in db/db mice.

The present invention has been described through the above examples. It should be understood that the above examples are for illustrative purposes and not intended to limit the present invention to the described embodiments. Additionally, those skilled in the art will understand that the present invention is not limited to the above examples, and various modifications and variations can be made based on the teachings of the present invention, which fall within the scope of protection required by the accompanying claims and their equivalents.

### List of Sequence

SEQ ID NO.1:
   **A14E, B16H, B25H, chain A of desB30 human insulin:** 
Gly Ile Val Glu Gln Cys Cys Thr Ser Ile Cys Ser Leu Glu Gln Leu Glu
Asn Tyr Cys Asn
SEQ ID NO.2:
   **A14E, B16H, B25H, chain B of desB30 human insulin:** 
Phe Val Asn Gln His Leu Cys Gly Ser His Leu Val Glu Ala Leu His
Leu Val Cys Gly Glu Arg Gly Phe His Tyr Thr Pro Lys
SEQ ID NO.3:
   **A14E, B16E, B25H, chain A of desB30 human insulin:** Gly Ile Ual Glu Gin Cys Cys Thr Ser Ile Cys Ser Leu Glu Gin Leu Glu Asn Tyr Cys Asn
SEQ ID NO.4:
   **A14E, B16E, B25H, chain B of desB30 human insulin:** 
Phe Val Asn Gln His Leu Cys Gly Ser His Leu Val Glu Ala Leu Glu
Leu Val Cys Gly Glu Arg Gly Phe His Tyr Thr Pro Lys
SEQ ID NO. 5:
   GLP-1-(7-37) peptide 
His Ala Glu Gly Thr Phe Thr Ser Asp Val Ser Ser Tyr Leu Glu Gly Gln
Ala Ala Lys Glu Phe Ile Ala Trp Leu Val Lys Gly Arg Gly
SEQ ID NO.6:
   [Gly8, Arg34]GLP-1-(7-37) peptide 
His Gly Glu Gly Thr Phe Thr Ser Asp Val Ser Ser Tyr Leu Glu Gly Gln
Ala Ala Lys Glu Phe Ile Ala Trp Leu Val Arg Gly Arg Gly
SEQ ID NO.7:
   [Arg34]GLP-1-(7-37) peptide

His Ala Glu Gly Thr Phe Thr Ser Asp Val Ser Ser Tyr Leu Glu Gly Gln
Ala Ala Lys Glu Phe Ile Ala Trp Leu Val Arg Gly Arg Gly

## Claims

1. An acylated insulin of formula (A), or a pharmaceutically acceptable salt, amide or ester thereof:
III-(II)ₘ-(I)ₙ-ins (A), wherein,
ins is an insulin parent of the acylated insulin, and III-(II)ₘ-(I)ₙ is an acyl moiety of the acylated insulin;
the insulin parent is A14E, B16H, B25H, desB30 human insulin, or A14E, B16E, B25H, desB30 human insulin, and the acyl moiety is linked to the ε amino group of the lysine residue at position B29 of the insulin parent;
I is a neutral, alkylene glycol-containing amino acid residue;
II is an acidic amino acid residue;
III is a fatty diacid containing 22, 23, 24, 25, or 26 carbon atoms, wherein formally, a hydroxyl group has been removed from one of the carboxyl groups of the fatty diacid;
III, II, and I are linked by an amide bond, and the order of II and I presented in the formula (A) can be interchanged independently;
m is 1, 2, 3, 4 or 5; and
n is an integer of 3 or 4.

2. The acylated insulin according to claim 1, wherein,
I is: -HN-(CH₂)₂-O-(CH₂)₂-O-CH₂-CO-, -HN-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₃-O-(CH₂)₄-O-(CH₂)₃-NH-CO-, -HN-(CH₂)₃-O-(CH₂)₄-O-(CH₂)₃-NH-CO-CH₂-O-CH₂-CO-, -HN-(CH₂)₃-O-(CH₂)₄-O-(CH₂)₃-NH-CO-(CH₂)₂-CO-, -HN-(CH₂)₂-O-(CH₂)₂-O-CH₂-CO-CH₂-O-CH₂-CO-, -HN-(CH₂)₃-O-(CH₂)₂-O-(CH₂)₂-O-(CH₂)₃-NH-CO-(CH₂)₂-CO-, -HN-(CH₂)₃-O-(CH₂)₂-O-(CH₂)₂-O-(CH₂)₃-NH-CO-CH₂-O-CH₂-CO-, -HN-(CH₂)₂-O-(CH₂)₂-O-(CH₂)₂-NH-CO-(CH₂)₂-CO-, -HN-(CH₂)₂-O-(CH₂)₂-O-(CH₂)₂-NH-CO-CH₂-O-CH₂-CO-, -HN-(CH₂)₃-O-(CH₂)₂-O-(CH₂)₂-O-(CH₂)₃-NH-CO-CH₂-O-CH₂-CO-, -HN-(CH₂)₃-O-(CH₂)₃-O-CH₂-CO-, or -HN-(CH₂)₄-O-(CH₂)₄-O-CH₂-CO-; preferably I is -HN-(CH₂)₂-O-(CH₂)₂-O-CH₂-CO-; and/or
II is an amino acid residue selected from the group consisting of γGlu, αGlu, βAsp, αAsp, γ-D-Glu, α-D-Glu, β-D-Asp and α-D-Asp, preferably, II is γGlu; and/or
III is a fatty diacid containing 22, 23 or 24 carbon atoms, wherein formally a hydroxyl group has been removed from one of the carboxyl groups of the fatty diacid; preferably III is HOOC-(CH₂)₂₀-CO-, HOOC-(CH₂)₂₁-CO-, or HOOC-(CH₂)₂₂-CO-; preferably, III is HOOC-(CH₂)₂₀-CO-.

3. The acylated insulin according to claim 1 or 2, wherein,
n is 3; and/or
m is 1 or 2.

4. The acylated insulin according to any one of claims 1-3, wherein,
the acylated insulin is selected from the group consisting of the following insulins:
A14E, B16H, B25H, B29K(N(ε)-docosanedioyl-γGlu-3xOEG), desB30 human insulin; A14E, B16H, B25H, B29K(N(ε)-docosanedioyl-γGlu-4xOEG), desB30 human insulin; A14E, B16H, B25H, B29K(N(ε)-tricosanedioyl-γGlu-3xOEG), desB30 human insulin; A14E, B16H, B25H, B29K(N(ε)-tricosanedioyl-γGlu-4xOEG), desB30 human insulin; A14E, B16H, B25H, B29K(N(ε)-tetracosanedioyl-γGlu-3xOEG), desB30 human insulin; A14E, B16H, B25H, B29K (N(ε)-tetracosanedioyl-γGlu-4xOEG),
desB30 human insulin; A14E, B16E, B25H, B29K (N(ε)-docosanedioyl-γGlu-3xOEG), desB30 human insulin; A14E, B16E, B25H, B29K (N(ε)-docosanedioyl-γGlu-4xOEG), desB30 human insulin; A14E, B16E, B25H, B29K(N(ε)-tricosanedioyl-γGlu-3xOEG), desB30 human insulin; A14E, B16E, B25H, B29K (N(ε)-tricosanedioyl-γGlu-4xOEG), desB30 human insulin; A14E, B16E, B25H, B29K(N(ε)-tetracosanedioyl-γGlu-3xOEG),
desB30 human insulin; and A14E, B16E, B25H, B29K (N(ε)-tetracosanedioyl-γGlu-4xOEG), desB30 human insulin;
preferably, the acylated insulin is selected from the group consisting of the following insulins: A14E, B16H, B25H, B29K(N(ε)-docosanedioyl-γGlu-3xOEG), desB30 human insulin; A14E, B16H, B25H, B29K(N(ε)-docosanedioyl-γGlu-4xOEG), desB30 human insulin; A14E, B16E, B25H, B29K(N(ε)-docosanedioyl-γGlu-3xOEG), desB30 human insulin; and
A14E, B16E, B25H, B29K (N(ε)-docosanedioyl-γGlu-4xOEG), desB30 human insulin;
more preferably, the acylated insulin is A14E, B16H, B25H, B29K (N(ε)-docosanedioyl-γGlu-3xOEG), desB30 human insulin, or A14E, B16H, B25H, B29K (N(ε))-docosanedioyl-γGlu-4xOEG), desB30 human insulin.

5. A pharmaceutical composition comprising the acylated insulin according to any one of claims 1-4 or A14E, B16H, B25H, B29K (N(ε)-docosanedioyl-γGlu-2xOEG), desB30 human insulin, and one or more pharmaceutically acceptable excipients.

6. The pharmaceutical composition according to claim 5 comprising at least about 1.5 moles of zinc ions/6 moles of the acylated insulin; preferably comprising at least about 2.2 moles of zinc ions/6 moles of the acylated insulin; preferably comprising about 2.2-12 moles of zinc ions/6 moles of the acylated insulin; preferably comprising about 2.3-10 moles of zinc ions/6 moles of the acylated insulin; more preferably comprising about 2.3-5.6 moles of zinc ions/6 moles of the acylated insulin; more preferably comprising about 2.3-4.8 moles of zinc ions/6 moles of insulin; more preferably comprising about 2.3-3.7 moles of zinc ions/6 moles of the acylated insulin; more preferably comprising about 2.3-3 moles of zinc ions/6 moles of the acylated insulin; and/or
the pharmaceutical composition has a pH of about 6.5-8.5; preferably a pH of about 6.8-8.2; preferably a pH of about 7.0-8.2; preferably a pH of about 7.2-7.6; more preferably a pH of about 7.4 or about 7.6.

7. The pharmaceutical composition according to claim 5 or 6, wherein, the pharmaceutical composition further comprises glycerol, phenol, *m*-cresol, NaCl, Na₂HPO₄, and/or citric acid; preferably, the pharmaceutical composition further comprises glycerol, phenol, and NaCl; preferably, the pharmaceutical composition further comprises glycerol, phenol, *m*-cresol, and NaCl; preferably, the pharmaceutical composition further comprises glycerol, phenol, NaCl and Na₂HPO₄; preferably, the pharmaceutical composition further comprises glycerol, phenol, NaCl and citric acid; more preferably, the pharmaceutical composition further comprises glycerol, phenol, *m*-cresol, NaCl and Na₂HPO₄; more preferably, the pharmaceutical composition further comprises glycerol, phenol, *m*-cresol, NaCl and citric acid.

8. The pharmaceutical composition according to claim 7, wherein the content of glycerol is no more than about 2.5% (w/w), preferably no more than about 2% (w/w), preferably about 0.3% to about 2% (w/w), preferably about 0.5% to about 1.8% (w/w), preferably about 0.7% to about 1.8% (w/w), preferably about 1% to about 1.7% (w/w); and/or the content of phenol is about 15-80mM, preferably about 25-75mM, preferably about 30-70mM, preferably about 35-70mM, preferably about 45-70mM, preferably about 45-65mM; preferably about 45mM, about 46mM, about 47mM, about 48mM, about 49mM, about 50mM, about 51mM, about 52mM, about 53mM, about 54mM, about 55mM, about 56mM, about 57mM, about 58mM, about 59mM, about 60mM, about 61mM, about 62mM, about 63mM, about 64mM, or about 65mM; and/or the content of the *m*-cresol is about 0-35mM, preferably about 0-19mM, preferably about 0-15mM, preferably about 0mM, about 1mM, about 2mM, about 3mM, about 4mM, about 5mM, about 6mM, about 7mM, about 8mM, about 9mM, about 10mM, about 11mM, about 12mM, about 13mM, about 14mM, or about 15mM; and/or
the content of the NaCl is about 0-150mM, preferably about 5-120mM, preferably about 10-120mM, preferably about 10-100mM, preferably about 10-75mM, preferably about 10-50mM, preferably about 10-30mM, preferably about 10-20mM; and/or
the content of the Na₂HPO₄ is about 0-75mM, preferably about 5-60mM, preferably about 5-50mM, preferably about 5-25mM, preferably about 5-10mM; and/or
the content of the citric acid is about 0-2mg/ml, preferably about 0.1-1.5mg/ml, preferably about 0.2-1mg/ml, preferably about 0.25-0.875mg/ml, preferably about 0.25-0.5mg /ml; and/or the content of the acylated insulin is higher than about 0.6mM, preferably about 1.2-9.0mM, preferably about 1.2-8.4mM, preferably about 2.1-7.2mM, preferably about 2.1-6.0mM, preferably about 2.1-4.2mM, preferably about 2.1-3.6mM.

9. The pharmaceutical composition according to any one of claims 5-8, wherein the acylated insulin is selected from the group consisting of the following insulins: A14E, B16H, B25H, B29K (N(ε)-docosanedioyl-γGlu-3xOEG), desB30 human insulin; A14E, B16H, B25H, B29K (N(ε)-docosanedioyl-yGlu-4xOEG), desB30 human insulin; A14E, B16H, B25H, B29K (N(ε)-tricosanedioyl-γGlu-3xOEG), desB30 human insulin; A14E, B16H, B25H, B29K (N(ε)-tricosanedioyl-γGlu-4xOEG), desB30 human insulin; A14E, B16H, B25H, B29K (N(ε)-tetracosanedioyl-γGlu-3xOEG), desB30 human insulin; A14E, B16H, B25H, B29K (N(ε)-tetracosanedioyl-γGlu-4xOEG), desB30 human insulin; A14E, B16E, B25H, B29K (N(ε)-docosanedioyl-γGlu-3xOEG), desB30 human insulin; A14E, B16E, B25H, B29K (N(ε)-docosanedioyl-γGlu-4xOEG), desB30 human insulin; A14E, B16E, B25H, B29K (N(ε)- tricosanedioyl-yGlu-3xOEG), desB30 human insulin; A14E, B16E, B25H, B29K (N(ε)-tricosanedioyl-γGlu-4xOEG), desB30 human insulin; A14E, B16E, B25H, B29K (N(ε)-tetracosanedioyl-γGlu-3xOEG), desB30 human insulin; A14E, B16E, B25H, B29K (N(ε)-tetracosanedioyl-γGlu-4xOEG), desB30 human insulin; and A14E, B16H, B25H, B29K (N(ε)-docosanedioyl-γGlu-2xOEG), desB30 human insulin.

10. A pharmaceutical composition comprising about 2.1-4.2mM (preferably about 2.1-3.5mM, preferably about 2.1-2.8mM, about 2.1mM) of acylated insulin, about 1% to about 2% (preferably about 1.5%-1.7%, more preferably about 1.7%) (weight/weight) of glycerol, about 15mM-65mM (preferably about 30mM-60mM, more preferably about 45mM-60mM, more preferably about 45mM-55mM) of phenol, about 1.5-7.0 (preferably about 2.2-5.6, preferably about 2.3-4.8, preferably about 2.3-3.7, preferably about 2.3-3, more preferably about 2.3) moles of zinc ions/6 moles of acylated insulin, about 10-120mM (preferably about 20-50mM, more preferably about 20mM) of sodium chloride, about 0-25mM (preferably about 0-15mM, preferably about 0-10mM, more preferably about 10mM) of *m*-cresol, and having a pH of about 7.0-8.2 (preferably about 7.4); or
comprising about 2.1-4.2mM (preferably about 2.1-3.5mM, preferably about 2.1-2.8mM, preferably about 2.1mM) of acylated insulin, about 1% to about 2% (preferably about 1.5%-1.7%, more preferably about 1.7%) (weight/weight) of glycerol, about 15mM-65mM (preferably about 30mM-60mM, more preferably about 45mM-60mM, more preferably about 45mM-55mM) of phenol, about 0-25mM (preferably about 0-15mM, preferably about 0-10mM, more preferably about 10mM) of *m*-cresol, about 10-120mM (preferably about 20-50mM, more preferably about 20mM) of NaCl, about 1.5-7.0 (preferably about 2.2-5.6, preferably about 2.3-4.8, preferably about 2.3-3.7, preferably about 2.3-3, preferably about 2.3) moles of zinc ions/6 moles of acylated insulin, about 0.1-1.5mg/ml (preferably about 0.2-1mg/ml, more preferably about 0.5mg/ml) of citric acid, and having a pH of about 7.0-8.2 (preferably about 7.4); or comprising about 2.1-4.2mM (preferably about 2.1-3.5mM, preferably about 2.1-2.8mM, preferably about 2.1mM) of acylated insulin, about 1% to about 2% (preferably about 1.5%-1.7%, more preferably about 1.7%) (weight/weight) of glycerol, about 15mM-65mM (preferably about 30mM-60mM, more preferably about 45mM-60mM, more preferably about 45mM-55mM) of phenol, about 0-25mM (preferably about 0-15mM, preferably about 0-10mM, more preferably about 10mM) of *m*-cresol, about 10-120mM (preferably about 20-50mM, more preferably about 20mM) of NaCl, about 1.5-7.0 (preferably about 2.2-5.6, preferably about 2.3-4.8, preferably about 2.3-3.7, preferably about 2.3-3, preferably about 2.3) moles of zinc ions/6 moles of acylated insulin, about 2-40mM (preferably about 5-10mM, more preferably about 5mM) of disodium hydrogen phosphate, and having a pH of about 7.0-8.2 (preferably about 7.4);
preferably, the acylated insulin is A14E, B16H, B25H, B29K(N(ε)-docosanedioyl-yGlu-3xOEG), desB30 human insulin; A14E, B16H, B25H, B29K (N(ε)-docosanedioyl-γGlu-4xOEG), desB30 human insulin; A14E, B16E, B25H, B29K (N(ε)-docosanedioyl-γGlu-3xOEG), desB30 human insulin; A14E, B16H, B25H, B29K (N(ε)-docosanedioyl-γGlu-2xOEG), desB30 human insulin; or A14E, B16E, B25H, B29K (N(ε)-docosanedioyl-γGlu-4xOEG), desB30 human insulin.

11. A pharmaceutical composition comprising about 2.1mM of acylated insulin, about 1.7% (weight/weight) of glycerol, about 45mM of phenol, about 2.3 moles of zinc ions/6 moles of acylated insulin, about 20mM of sodium chloride, about 10mM of *m*-cresol, and having a pH of about 7.4; or
comprising about 2.1mM acylated insulin, about 1.7%(w/w) of glycerol, about 45mM of phenol, about 10mM of *m*-cresol, about 20mM of NaCl, about 2.3 moles of zinc ions/6 moles of acylated insulin, about 0.5mg/ml of citric acid, and having a pH of about 7.4; or
comprising about 2.1mM of acylated insulin, about 1.7% (w/w) of glycerol, about 45mM of phenol, about 10mM of *m*-cresol, about 20mM of NaCl,
about 2.3 moles of zinc ions/6 moles of acylated insulin, about 5mM of disodium hydrogen phosphate, and having a pH of about 7.4;
preferably, the acylated insulin is A14E, B16H, B25H, B29K (N(ε)-docosanedioyl-γGlu-3xOEG), desB30 human insulin; A14E, B16H, B25H, B29K(N(ε)-docosanedioyl-γGlu-4xOEG), desB30 human insulin; A14E, B16E, B25H, B29K (N(ε)-docosanedioyl-γGlu-3xOEG), desB30 human insulin; A14E, B16H, B25H, B29K(N(ε)-docosanedioyl-γGlu-2xOEG), desB30 human insulin; or A14E, B16E, B25H, B29K (N(ε)-docosanedioyl-γGlu-4xOEG), desB30 human insulin.

12. The pharmaceutical composition according to any one of claims 5-11, further comprising an insulinotropic GLP-1 compound; preferably, the pharmaceutical composition further comprises an insulinotropic GLP-1 compound selected from the group consisting of the following insulinotropic GLP-1 compounds:
*N-*ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl ][Gly8, Arg34]GLP-1-(7-37) peptide,
*N-*ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
*N-*ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N-*ε²⁶-[2-(2-[2-(4-[21-carboxyheneicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Gly8, Arg34]GLP-1-(7-37) peptide,
*N-*ε²⁶-(23-carboxytricosanoylamino)-4(*S*)-carboxybutanoyl-[Gly8, Arg34]GLP-1-(7-37) peptide,
*N-*ε²⁶-(19-carboxynonadecanoylamino)-4(*S*)-carboxybutanoyl-[Gly8, Arg34]GLP-1-(7-37) peptide,
*N*-ε²⁶-(21-carboxyheneicosanoylamino)-4(*S*)-carboxybutanoyl-[Gly8, Arg34]GLP-1-(7-37) peptide,
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Arg34]GLP-1-(7-37) peptide,
*N-*ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl ][Arg34]GLP-1-(7-37) peptide,
*N-*ε²⁶-[2-(2-[2-(4-[23-carboxytricosanoylamino]-4(*S*)-carboxybutanoylamino)ethoxy]ethoxy)acetyl][Arg34]GLP-1-(7-37) peptide,
*N-*ε²⁶-(23-carboxytricosanoylamino)-4(*S*)-carboxybutanoyl-[Arg34]GLP-1-(7-37) peptide,
N-ε²⁶-(19-carboxynonadecanoylamino)-4(*S*)-carboxybutanoyl-[Arg34]GLP-1-(7-37) peptide,
*N-*ε²⁶-(21-carboxyheneicosanoylamino)-4(*S*)-carboxybutanoyl-[Arg34]GLP-1-(7-37) peptide,
*N-*ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N-*ε²⁶-[2-(2-[2-(4-[20-carboxyeicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N-*ε²⁶-[2-(2-[2-(4-[22-carboxydocosanoylamino]-4(*S*)-carboxybutanoylamino)ethoxy]ethoxy)acetyl][Gly8, Arg34]GLP-1-(7-37) peptide,
*N*-ε²⁶-(20-carboxyeicosanoylamino)-4(*S*)-carboxybutanoyl-[Gly8, Arg34]GLP-1-(7-37) peptide,
*N-*ε²⁶-(22-carboxydocosanoylamino)-4(*S*)-carboxybutanoyl-[Gly8, Arg34]GLP-1-(7-37) peptide,
*N-*ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl ][Arg34]GLP-1-(7-37) peptide,
*N-*ε²⁶-[2-(2-[2-(4-[20-carboxyeicosanoylamino]-4(*S*)-carboxybutanoylamino)ethoxy]ethoxy)acetyl][Arg34]GLP-1-(7-37) peptide,
*N-*ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl ][Arg34]GLP-1-(7-37) peptide,
*N-*ε²⁶-[2-(2-[2-(4-[22-carboxydocosanoylamino]-4(*S*)-carboxybutanoylamino)ethoxy]ethoxy)acetyl][Arg34]GLP-1-(7-37) peptide,
*N-*ε²⁶-(20-carboxyeicosanoylamino)-4(*S*)-carboxybutanoyl-[Arg34]GLP-1-(7-37) peptide,
*N-*ε²⁶-(22-carboxydocosanoylamino)-4(*S*)-carboxybutanoyl-[Arg34]GLP-1-(7-37) peptide,
*N*-ε²⁶-(17-carboxyheptadecanoylamino)-4(*S*)-carboxybutanoyl-Arg34]GLP-1-(7-37) peptide,
*N*-ε²⁶-(17-carboxyheptadecanoylamino)-4(*S*)-carboxybutanoyl-[Gly8, Arg34]GLP-1-(7-37) peptide,
*N-*ε²⁶-[2-(2-[2-(2-[2-(2-[4-(17-carboxyheptadecanoylamino)-4(*S*)-carboxybutanoylamino]ethoxy)ethoxy]acetylamino)ethoxy]ethoxy)acetyl ][Aib8, Arg34]GLP-1-(7-37) peptide,
and *N*-ε²⁶-[2-(2-[2-(2-[2-(2-[4-(17-carboxyheptadecanoylamino)-4(*S*)-carboxybutanoylamino]ethoxy)ethoxy]acetylamino)ethoxy]ethoxy)acetyl ][Gly8, Arg34]GLP-1-(7-37) peptide.

13. The pharmaceutical composition according to claim 12, wherein,
the molar ratio of the insulinotropic GLP-1 compound to the acylated insulin is at least about 1:100, preferably at least about 3:100, preferably at least about 5:100, preferably at least about 8:100, preferably at least about (3:100)-(100:100), preferably about (5:100)-(80:100), preferably about (8:100)-(50:100), preferably about (10:100)-(50:100), preferably about (13:100)-(50:100), preferably about (13:100)-(40:100), preferably about (13:100)-(35: 100), preferably about (13:100)-(27:100), preferably about (13:100)-(20:100).

14. The acylated insulin according to any one of claims 1-4 or the pharmaceutical composition according to any one of claims 5-13 for use as a medicament.

15. The acylated insulin according to any one of claims 1-4 or the pharmaceutical composition according to any one of claims 5-13 for use as a medicament for treating diabetes.

16. Use of the acylated insulin according to any one of claims 1-4 or the pharmaceutical composition according to any one of claims 5-13 in the manufacture of a medicament for treating diabetes.

17. A method for treating diabetes, comprising administering a therapeutically effective amount of the acylated insulin according to any one of claims 1-4 or the pharmaceutical composition according to any one of claims 5-13 to a subject in need thereof.

18. A method for treating diabetes, comprising administering a therapeutically effective amount of the acylated insulin of formula (A), or the pharmaceutically acceptable salt, amide or ester thereof, to a subject in need thereof, wherein the acylated insulin is administered to the subject every 4 days or less frequency; preferably, the acylated insulin is administered to the subject every 5 days or less frequency; preferably, the acylated insulin is administered to the subject every 6 days or less frequency; preferably, the acylated insulin is administered to the subject every 7 days or less frequency; preferably, the acylated insulin is administered to the subject every 8 days or less frequency; preferably, the acylated insulin is administered to the subject every 9 days or less frequency; preferably, the acylated insulin is administered to the subject every 10 days or less frequency; preferably, the acylated insulin is administered to the subject every 2 weeks or more frequency:
III-(II)ₘ-(I)ₙ-ins (A),
wherein,
ins is the insulin parent of the acylated insulin, and III-(II)ₘ-(I)ₙ is the acyl moiety of the acylated insulin;
the insulin parent is A14E, B16H, B25H, desB30 human insulin or A14E, B16E, B25H, desB30 human insulin, and the acyl moiety is linked to the ε amino group of the lysine residue at position B29 of the insulin parent;
I is a neutral, alkylene glycol-containing amino acid residue;
II is an acidic amino acid residue;
III is a fatty diacid comprising 22, 23, 24, 25, or 26 carbon atoms, wherein formally, a hydroxyl group has been removed from one of the carboxyl groups in the fatty diacid;
III, II, and I are linked by an amide bond, and the order of II and I presented in formula (A) can be interchanged independently;
m is 1, 2, 3, 4 or 5, and n is an integer of 3 or 4.

19. The method according to claim 18, wherein,
I is: -HN-(CH₂)₂-O-(CH₂)₂-O-CH₂-CO-, -HN-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₃-O-(CH₂)₄-O-(CH₂)₃-NH-CO-, -HN-(CH₂)₃-O-(CH₂)₄-O-(CH₂)₃-NH-CO-CH₂-O-CH₂-CO-, -HN-(CH₂)₃-O-(CH₂)₄-O-(CH₂)₃-NH-CO-(CH₂)₂-CO-, -HN-(CH₂)₂-O-(CH₂)₂-O-CH₂-CO-CH₂-O-CH₂-CO-, -HN-(CH₂)₃-O-(CH₂)₂-O-(CH₂)₂-O-(CH₂)₃-NH-CO-(CH₂)₂-CO-, -HN-(CH₂)₃-O-(CH₂)₂-O-(CH₂)₂-O-(CH₂)₃-NH-CO-CH₂-O-CH₂-CO-, -HN-(CH₂)₂-O-(CH₂)₂-O-(CH₂)₂-NH-CO-(CH₂)₂-CO-, -HN-(CH₂)₂-O-(CH₂)₂-O-(CH₂)₂-NH-CO-CH₂-O-CH₂-CO-, -HN-(CH₂)₃-O-(CH₂)₂-O-(CH₂)₂-O-(CH₂)₃-NH-CO-CH₂-O-CH₂-CO-, -HN-(CH₂)₃-O-(CH₂)₃-O-CH₂-CO-, or -HN-(CH₂)₄-O-(CH₂)₄-O-CH₂-CO-; preferably I is -HN-(CH₂)₂-O-(CH₂)₂-O-CH₂-CO-; and/or
II is an amino acid residue selected from the group consisting of yGlu, αGlu, βAsp, αAsp, γ-D-Glu, α-D-Glu, β-D-Asp and α-D-Asp, preferably, II is γGlu; and /or
III is a fatty diacid containing 22, 23 or 24 carbon atoms, wherein formally a hydroxyl group has been removed from one of the carboxyl groups of the fatty diacid; preferably III is HOOC-(CH₂)₂₀-CO-, HOOC-(CH₂)₂₁-CO-, or HOOC-(CH₂)₂₂-CO-; preferably, III is HOOC-(CH₂)₂₀-CO-.

20. The method according to claim 18 or 19, wherein,
n is 3; and/or m is 1 or 2.

21. The method according to any one of claims 18-20, wherein,
the acylated insulin is selected from the group consisting of the following insulins:
A14E, B16H, B25H, B29K (N(ε)-docosanedioyl-γGlu-3xOEG), desB30 human insulin; A14E, B16H, B25H, B29K (N(ε)-docosanedioyl-γGlu-4xOEG), desB30 human insulin; A14E, B16H, B25H, B29K (N(ε)-tricosanedioyl-γGlu-SxOEG), desB30 human insulin; A14E, B16H, B25H,
B29K (N(ε)-tricosanedioyl-γGlu-4xOEG), desB30 human insulin; A14E, B16H, B25H, B29K (N(ε)-tetracosanedioyl-γGlu-3xOEG), desB30 human insulin; A14E, B16H, B25H, B29K (N(ε)-tetracosanedioyl-γGlu-4xOEG),
desB30 human insulin; A14E, B16E, B25H, B29K (N(ε)-docosanedioyl-γGlu-3xOEG), desB30 human insulin; A14E, B16E, B25H, B29K (N(ε)-docosanedioyl-γGlu-4xOEG), desB30 human insulin; A14E, B16E, B25H,
B29K (N(ε)- tricosanedioyl-γGlu-3xOEG), desB30 human insulin; A14E, B16E, B25H, B29K (N(ε)-tricosanedioyl-γGlu-4xOEG), desB30 human insulin ; A14E, B16E, B25H, B29K (N(ε)-tetracosanedioyl-γGlu-3xOEG),
desB30 human insulin; and A14E, B16E, B25H, B29K (N(ε)-tetracosanedioyl-γGlu-4xOEG), desB30 human insulin;
preferably, the acylated insulin is selected from the group consisting of the following insulins: A14E, B16H, B25H, B29K (N(ε)-docosanedioyl-γGlu-3xOEG), desB30 human insulin; A14E, B16H, B25H, B29K (N(ε)-docosanedioyl-γGlu-4xOEG), desB30 human insulin; A14E, B16E, B25H,
B29K (N(ε)-docosanedioyl-γGlu-3xOEG), desB30 human insulin; and
A14E, B16E, B25H, B29K (N(ε)-docosanedioyl-γGlu-4xOEG), desB30 human insulin;
more preferably, the acylated insulin is A14E, B16H, B25H, B29K (N(ε)-docosanedioyl-γGlu-3xOEG), desB30 human insulin, or A14E, B16H, B25H, B29K (N(ε))-docosanedioyl-γGlu-4xOEG), desB30 human insulin.

22. The method according to any one of claims 18-21, wherein,
the diabetes is type 1 diabetes or type 2 diabetes.
